(19) Europäisches Patentamt
European Patent Office
Office européen des brevets

(11) **EP 3 975 110 A1**

(12) **EUROPEAN PATENT APPLICATION**

(43) Date of publication:
**30.03.2022 Bulletin 2022/13**

(21) Application number: **20198551.2**

(22) Date of filing: **25.09.2020**

(51) International Patent Classification (IPC):
***G06T 7/00*** (2017.01)

(52) Cooperative Patent Classification (CPC):
**G06T 7/0012;** G06T 2207/10056;
G06T 2207/20021; G06T 2207/20081;
G06T 2207/20084; G06T 2207/30024

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Panakeia Technologies Limited Cambridge CB1 2LA (GB)**

(72) Inventors:
• **RAHARJA, Muhammad Pandu Cambridge, CB1 2LA (GB)**

• **ARSLAN, Salim Sedat Cambridge, CB1 2LA (GB)**
• **DE MAUPEOU D'ABLEIGES, Sixte Gabriel Marie Gilles Cambridge, CB1 2LA (GB)**
• **DAYAN, Benjamin Li Cambridge, CB1 2LA (GB)**

(74) Representative: **Sibley, Lara Ann Marks & Clerk LLP 15 Fetter Lane London EC4A 1BW (GB)**

(54) **A METHOD OF PROCESSING AN IMAGE OF TISSUE AND A SYSTEM FOR PROCESSING AN IMAGE OF TISSUE**

(57)     A computer implemented method of processing an image of tissue, comprising:
obtaining a first set of image portions from an input image of tissue;
selecting a second set of one or more image portions from the first set of image portions, the selecting comprising inputting image data of an image portion from the first set into a first trained model comprising a first convolutional neural network, the first trained model generating an indication of whether the image portion is associated with a biomarker; and
determining an indication of whether the input image is associated with the biomarker from the second set of one or more image portions.

Figure 5(a)

**Description**

FIELD

The present invention relates to a computer-implemented method of processing an image of tissue and a system for processing an image of tissue.

BACKGROUND

A biomarker is a naturally occurring molecule, gene, or characteristic by which a particular pathological or physiological process, disease, diagnosis, therapy or prognosis can be identified. For example, modern cancer diagnosis and treatment may rely on understanding the specific molecular profile of the cancer, and patient in general. The molecular profile includes one or more molecular biomarkers. The molecular profile may be used to inform various procedures, including hormone therapies, immunotherapies and targeted drugs treatment.

Various medically relevant biomarkers - for example diagnostics, therapeutics and/or prognostics markers, such as: mutation status, receptor status, copy number variations and others - are tested through means of genetic, transcriptomic and immunological assay, in order to determine how well a patient would respond to certain therapies. Such tests are conducted on human samples called biopsies, which may be in liquid or solid forms. Such testing may take, depending on the type of test and sample, anywhere between 1 and 30 days and is prone to procedural error. The results of such procedures are then analysed by experts - pathologist for tissue biopsy, hematologist for liquid biopsy, cytopathologist for cytology samples, geneticist for genetic/transcriptomic assay etc. This again may be time-intensive and highly vulnerable to human error. There is a continuing need to improve the reliability, economy and speed of detection of such biomarkers.

BRIEF DESCRIPTION OF FIGURES

Systems and methods in accordance with non-limiting embodiments will now be described with reference to the accompanying figures in which:

Figure 1 shows a schematic illustration of a system in accordance with an embodiment;

Figure 2(a) is a schematic illustration of a method of processing an image of tissue in accordance with an embodiment

Figure 2(b) is an example of an image of a histological section stained with hematoxylin and eosin;

Figure 3(a) shows a schematic illustration of an input image, which is an image of a histological section stained with hematoxylin and eosin, and an output, which is a first set of image portions;

Figure 3(b) shows a schematic illustration of an image pre-processing step used in a method in accordance with an embodiment;

Figure 3(c) shows a schematic illustration of an example segmentation model based on a CNN used in the image pre-processing step;

Figure 3(d) shows a schematic illustration of a filter which performs a dilated convolution;

Figure 3(e) is a schematic illustration of an example method of training a cell segmentation model;

Figure 4 shows a schematic illustration of a method of processing an image of tissue according to an embodiment, in which a pooling operator is used;

Figure 5(a) shows a schematic illustration of a method of processing an image of tissue according to an embodiment;

Figure 5(b) shows a schematic illustration of an example recurrent neural network based on a Long Short Term Memory structure, which may be used in the method described in relation to Figure 5(a);

Figure 5(c) shows a schematic illustration of an example first convolutional neural network which may be used in the method described in relation to Figure 5(a);

Figure 6(a) shows a schematic illustration of a method in accordance with an alternative embodiment;

Figure 6(b) shows a schematic illustration of an example attention module structure that may be used in the method of Figure 6(a);

Figure 7 shows a schematic illustration of a method in accordance with an alternative embodiment;

Figure 8 shows a schematic illustration of an example cancer diagnosis pipeline;

Figure 9 shows an example diagnosis pipeline using automatic profiling of one or more biomarkers with a method in accordance with an embodiment;

Figure 10 shows a schematic illustration of a method in accordance with an alternative embodiment;

Figure 11 shows a schematic illustration of a method of training in accordance with an embodiment.

DETAILED DESCRIPTION

According to an embodiment, there is provided a computer implemented method of processing an image of tissue, comprising:

obtaining a first set of image portions from an input image of tissue;
selecting a second set of one or more image portions from the first set of image portions, the selecting comprising inputting image data of an image portion from the first set into a first trained model comprising a first convolutional neural network, the first trained model generating an indication of whether the image portion is associated with a biomarker; and
determining an indication of whether the input image is associated with the biomarker from the second set of one or more image portions.

In an embodiment, the second set comprises two or more image portions, and wherein the determining comprises inputting first data corresponding to the second set of one or more image portions into a second trained model. The second trained model may comprise a neural network. The second trained model may comprise a recurrent neural network. The second trained model may additionally or alternatively comprise an attention mechanism.

In an embodiment, the second trained model may comprise a recurrent neural network and an attention mechanism, and wherein determining an indication of whether the input image is associated with the biomarker from the second set of image portions comprises: inputting the first data for each image portion in the second set into the attention mechanism, wherein the attention mechanism is configured to output an indication of the importance of each image portion; selecting a third set of image portions based on the indication of the importance of each image portion; and for each image portion in the third set, inputting the first data into the recurrent neural network, the recurrent neural network generating the indication of whether the input image is associated with the biomarker.

In an embodiment, the indication of whether the image portion is associated with the biomarker is a probability that the image portion is associated with the biomarker, wherein selecting the second set comprises selecting the k image portions having the highest probability, wherein k is a pre-defined integer greater than 1.

In an embodiment, the first convolutional neural network comprises a first portion comprising at least one convolutional layer and a second portion, wherein the second portion takes as input a one dimensional vector; wherein determining the indication of whether the input image is associated with the biomarker from the second set of image portions further comprises: generating the first data for each of the second set of image portions, generating the first data for an image portion comprising inputting the image data of the image portion into the first portion of the first convolutional neural network.

In an embodiment, selecting a fourth set of one or more image portions from the first set of image portions, the selecting comprising inputting image data of an image portion from the first set into a third trained model comprising a second convolutional neural network; the third trained model generating an indication of whether the image portion is not associated with the biomarker; and wherein the indication of whether the input image is associated with the biomarker is determined from the fourth set of one or more image portions and the second set of one or more image portions.

In an embodiment, the biomarker is a cancer biomarker and wherein obtaining the first set of image portions from an input image of tissue comprises:

splitting the input image of tissue into image portions;

inputting image data of an image portion into a fifth trained model, the fifth trained model generating an indication of whether the image portion is associated with cancer tissue; and

selecting the first set of image portions based on the indication of whether the image portion is associated with cancer tissue.

In an embodiment, the biomarker is a molecular biomarker.

According to a second aspect, there is provided a system for processing an image of tissue, comprising:

an input configured to receive an input image of tissue;
an output configured to output an indication of whether the input image is associated with a biomarker
one or more processors, configured to:

obtain a first set of image portions from an input image of tissue received by way of the input;
select a second set of one or more image portions from the first set of image portions, the selecting comprising inputting image data of an image portion from the first set into a first trained model comprising a first convolutional neural network, the first trained model generating an indication of whether the image portion is associated with a biomarker;

determine an indication of whether the input image is associated with the biomarker from the second set of one or more image portions; and
output the indication by way of the output.

According to a third aspect, there is provided a computer implemented method of training, comprising:

obtaining a first set of image portions from an input image of tissue;
inputting image data of an image portion from the first set into a first model comprising a first convolutional neural network, the first model generating an indication of whether the image portion is associated with a biomarker;
adapting the first model based on a label associated with the input image of tissue indicating whether the input image is associated with the biomarker.

In an embodiment, the method further comprises:

selecting a second set of one or more image portions from the first set of image portions based on the indication of whether the image portion is associated with a biomarker;
determining an indication of whether the input image is associated with the biomarker from the second set of one or more image portions by inputting first data corresponding to the second set of image portions into a second model, and wherein the method further comprises adapting the second model based on the label associated with the input image of tissue indicating whether the input image is associated with the biomarker.

In an embodiment, the method further comprises adapting the first model again based on the label associated with the input image of tissue indicating whether the input image is associated with the biomarker.

In an embodiment, the first convolutional neural network comprises a first portion comprising at least one convolutional layer and a second portion, wherein the second portion takes as input a one dimensional vector; wherein determining the indication of whether the input image is associated with the biomarker from the second set of image portions further comprises: generating the first data for each of the second set of image portions, generating the first data for an image portion comprising inputting the image data of the image portion into the first portion of the first convolutional neural network.

In an embodiment, the method comprises:

obtaining the first set of image portions from a first input image of tissue associated with a label indicating the input image is associated with the biomarker;
selecting a second set of one or more image portions from the first set of image portions based on the indication of whether the image portion is associated with a biomarker;
obtaining a further set of image portions from a second input image of tissue associated with a label indicating the input image is not associated with the biomarker;
selecting a fourth set of one or more image portions from the further set of image portions based on the indication of whether the image portion is associated with a biomarker;
generating the first data for the second set of image portions, generating the first data for an image portion comprising inputting the image data of the image portion into the first portion of the first convolutional neural network;
generating the first data for the fourth set of image portions, generating the first data for an image portion comprising inputting the image data of the image portion into the first portion of the first convolutional neural network;
determining a distance measure between the first data for the second set of image portions and the first data for the fourth set of image portions;
adapting the first model based on the different measure.

According to a fourth aspect, there is provided a system comprising a first model and a second model trained according to the above methods.

According to a fifth aspect, there is provided a carrier medium comprising computer readable code configured to cause a computer to perform the above methods. The methods are computer-implemented methods. Since some methods in accordance with embodiments can be implemented by software, some embodiments encompass computer code provided to a general purpose computer on any suitable carrier medium. The carrier medium can comprise any storage medium such as a floppy disk, a CD ROM, a magnetic device or a programmable memory device, or any transient medium such as any signal e.g. an electrical, optical or microwave signal. The carrier medium may comprise a non-transitory computer readable storage medium.

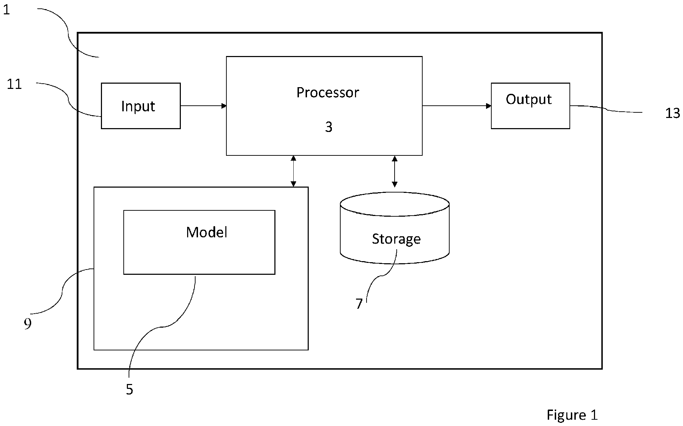

Figure 1 shows a schematic illustration of a system 1 in accordance with an embodiment. The system 1 comprises an input 11, a processor 3, a working memory 9, an output 13, and storage 7. The system 1 takes input image data and generates an output. The output may comprise diagnostic information. In particular, the output may be an indication of whether the input image is associated with a biomarker.

The system 1 may be a computing system, for example an end-user system or a server. In an embodiment, the system comprises a graphical processing unit (GPU) and a general central processing unit (CPU). Various operations described in relation to the methods below are implemented by the GPU, whereas other operations are implemented by the CPU. For example, matrix operations may be performed by the GPU.

The processor 3 is coupled to the storage 7 and accesses the working memory 9. The processor 3 may comprise logic circuitry that responds to and processes the instructions in code stored in the working memory 9.

A computer program 5 is stored in non-volatile memory. The non-volatile memory 9 is accessed by the processor 3 and the stored code 5 is retrieved and executed by the processor 3. In particular, when executed, computer program code 5 embodying the methods described below is represented as a software product stored in the working memory 9. Execution of the code 5 by the processor 3 will cause embodiments as described herein to be implemented.

The processor 3 also accesses the input module 11 and the output module 13. The input and output modules or interfaces 11, 13 may be a single component or may be divided into a separate input interface 11 and a separate output interface 13.

The input module 11 is connected to an input 15 for receiving the image data. The input 15 may be a receiver for receiving data from an external storage medium or through a communication network. Alternatively, the input 15 may comprise hardware such as an image capturing apparatus. Alternatively, the input 15 may read data from a stored image file, which may be stored on the system or on a separate storage medium such as a floppy disk, a CD ROM, a magnetic device or a programmable memory device.

Connected to the output module 13 is output 17. The output 17 may comprise hardware, such as a visual display. Alternatively, the output may be a transmitter for transmitting data to an external storage medium or through a communication network. Alternatively, the output 17 may write data in a stored image file, which may be stored on the system or on a separate storage medium such as a floppy disk, a CD ROM, a magnetic device or a programmable memory device.

The storage 7 is communicatively coupled with the processor 3. The storage 7 may contain data that is used by the code 5 when executed by the processor 3. As illustrated, the storage 7 is local memory that is contained in the device. Alternatively however, the storage 7 may be wholly or partly located remotely, for example, using cloud based memory that can be accessed remotely via a communication network (such as the Internet). The code 5 is also stored in the storage 7. The code 5 is placed in working memory 9 when executed.

The system 1 may be located in a common system with hardware for inputting and outputting data. Alternatively, the system 1 may be a remote system 1, which receives image data transmitted from a separate unit (for example an image capturing device), and transmits output data to another separate unit (for example a user computer comprising a screen). For example, the system may be implemented on a cloud computing system, which receives and transmits data. Although in the described system, a single processor 3 located in a device is used, the system may comprise two or more processors, which may be located in the same system or located remotely, being configured to perform different parts of the processing and transmit data between them.

Usual procedures for the loading of software into memory and the storage of data in the storage unit 7 apply. The code 5 can be embedded in original equipment, or can be provided, as a whole or in part, after manufacture. For instance, the code can be introduced, as a whole, as a computer program product, which may be in the form of a download, or can be introduced via a computer program storage medium, such as an optical disk. Alternatively, modifications to existing dialogue manager software can be made by an update, or plug-in, to provide features of the described embodiments.

While it will be appreciated that the described embodiments are applicable to any computing system, the example computing system illustrated in Figure 1 provides means capable of putting an embodiment, as described herein, into effect.

In use, the system 1 receives image data through data input 11. The program 5, executed on processor 3, outputs data through the output 13 in the manner which will be described with reference to the following figures. The processor 3 may comprise logic circuitry that responds to and processes the program instructions.

Where the system 1 is integrated in a hospital or healthcare system, the system 1 may also access information stored on the hospital or healthcare system, such as patient information or patient treatment history. Where the system 1 is implemented as a web service (i.e. it is not integrated in a hospital/healthcare system), an image is uploaded and analysed. Other data such as patient information may be uploaded together with the image. The analysis output may be stored in a database and/or transmitted back to the user system. A hybrid approach can be implemented in which a histopathologist uploads a set of images and these are analysed within a hospital or healthcare integrated system.

In one implementation, input image data is input through a user interface. A Representational State Transfer (REST) web service operates on the system. The REST service operates to re-construct pixel data from the transmitted data received from the user, and also manage transfer of data to and from the analysis record for example. These operations are performed on a CPU. The user interface and REST service may also operate to receive user input selecting options for implementing the system, for example which models to use, which information to output. The output data and the data input is stored in cloud based storage, referred to as the analysis record. The system is implemented on a cloud computing system, which receives image data and provides output data to cloud storage.

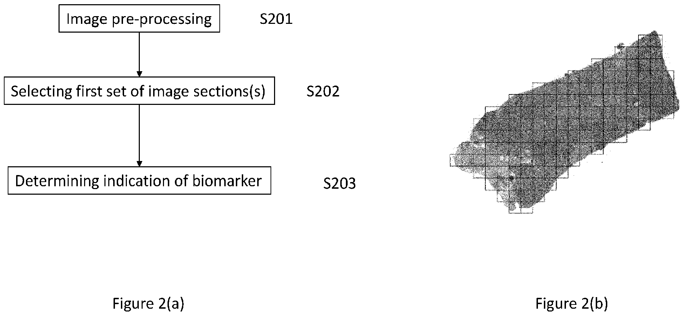

Figure 2(a) is a schematic illustration of a method of processing an image of tissue in accordance with an embodiment. The method may be implemented on a system such as described in relation to Figure 1.

The method takes as input image data I comprising a plurality of pixels. The input image data I comprises pixel data. In the below description, the pixel data is red-green-blue (of dimension height x width x 3), however the pixel data may alternatively be grayscale (of dimension height x width x 1) for example. The input image data comprises a first number of pixels, where the first number is equal to height x width. The image data may initially be acquired using a microscope mounted digital camera capturing images of tissue (also referred to as a histological section).

In a specific example described herein, the input I comprises an image of a histological section stained with hematoxylin and eosin stain. An example of an image of a histological section stained with hematoxylin and eosin stain is shown in Figure 2(b). A grid is overlaid on the image in this figure. A whole slide image (WSI) scanner may scan an entire tissue slice, resulting in an image of a histological section stained with hematoxylin and eosin stain comprising around 60 000 pixels height by 60 000 pixels width for example.

However, various types of tissue images obtained using various methods may be processed using the described method. For example, alternatively, an image of a histological section which has undergone Immunohistochemistry (IHC) staining may be taken as input. IHC staining involves selectively identifying antigens in cells of a tissue section. Antibodies bind specifically to antigens in biological tissues. The staining allows visualisation of an antibody-antigen interaction. For example, using chromogenic immunohistochemistry (CIH), an antibody is conjugated to an enzyme that can catalyse a colour-producing reaction.

The method determines an indication of whether the input image is associated with a specific biomarker. A biomarker is a naturally occurring molecule, gene, or characteristic by which a particular pathological or physiological process, disease, diagnosis, therapy or prognosis can be identified. In a specific example described herein, the biomarker is a cancer biomarker, i.e. a naturally occurring molecule, gene, or characteristic by which a particular type of cancer, or a particularly effective cancer treatment, can be identified. Furthermore, in the example described herein, the biomarker is a molecular biomarker. The biomarker may be a molecule or a characteristic associated with one of one or more molecules, such as an amount of a particular molecule for example. In some cases, the biomarker is a molecule associated with a specific cancer treatment. The biomarker may be a clinically actionable genetic alteration. Determining the presence of a biomarker from image data is more challenging than, for example, tumour detection from image data where morphological differences between normal and cancer cells are to be expected.

By understanding the specific molecular profile of the cancer and/or the patient in general, various procedures conducted against cancer including hormone therapies, immunotherapies or targeted drugs treatments amongst others can be informed. Various medically relevant biomarkers, including any of diagnostics, therapeutics or prognostics markers, including mutation status, receptor status, or copy number variations amongst others, may be identified to determine how well a patient would respond to certain therapies. Mutation status, receptor status, or copy number variations are examples of molecular biomarkers. For example, in some cases the molecular biomarker may be a protein expression level.

For example, the specific biomarker may be the Estrogen Receptor (ER), Progesterone Receptor (PR) or Human Epidermal Growth Factor Receptor (HER2). These pillar biomarkers are specific for breast cancer. They are the most important biomarkers for prognosis in breast cancer, and lie on the basis of targeted therapies. ER and HER2 are most commonly associated with cancer treatments Tamoxifen and Herceptin respectively. A patient may be tested for these two biomarkers to determine suitability for these treatments. The method described herein may be used to determine an indication of whether the input image is associated with the ER biomarker. This indication may be a probability for example. The method described herein may alternatively be used to determine an indication of whether the input image is associated with the HER2 biomarker. The method described herein may alternatively be used to determine an indication of whether the input image is associated with the PR biomarker. The specific biomarker may alternatively be EGFR, which is associated with lung adenocarcinoma. The specific biomarker may alternatively be MSI, which is associated with colon adenocarcinoma.

Various molecular biomarkers may be used to classify certain cancers into categories, such as breast or colorectal. For instance breast cancer has five different molecular "subtypes", each determined based on the statuses of ER, PR and HER2. For example, if ER, PR and HER2 are all negative, the molecular sub-type is "basal-like". Thus by determining the presence or absence of multiple molecular biomarkers, a molecular sub-type may be predicted. A "molecular subtype" is a way of categorising a particular type of cancer based on the presence or absence or, in some cases, level of one or a set of biomarkers.

The method may be used to detect various other biomarkers. For example, the antigen Ki-67 is also increasingly being tested as a marker for cell proliferation indicating cancer aggressiveness. The specific biomarker may thus alternatively be Ki-67. A labelling index based on IHC-staining of the Ki67 nuclear antigen can be used with other IHC markers as an alternative to mitotic counts in grading schemes when assessing tumour proliferation of HER2- and ER+ breast cancer for example. It may provide additional information for therapeutic decisions, such as any requirement for adjuvant chemotherapy. In various studies it was shown to be a powerful predictor of survival. For example, PREDICT is an online tool that shows how different treatments for early invasive breast cancer might improve survival rates after surgery. The PREDICT model performance was improved with the involvement of Ki67 as a prognostic marker. A manual scoring method to interpret IHC-stained Ki67 slides includes counting the invasive cells in a randomly selected region of interest, such as at the periphery of the tumor, and determining the percentage of Ki67 staining with respect to all invasive tumour cells. Similar to conventional molecular profiling techniques described above, this process is labour-intensive, prone to human errors, and open to inter-/intra observer. By predicting the Ki67 index from H&E images for example, such a process may be made shorter and the accuracy potentially improved.

The example method described herein provides automatic profiling of a specific biomarker relevant for diagnostics, therapeutics and/or prognostics of cancer. The specific biomarker may be a mutation status, receptor status or copy number variations, amongst other examples. The profiling is performed from whole slide H&E images in this example, although other images may be used. The example method comprises applying a series of neural networks to identify correlations between cancer images and a biomarker. In the example described herein, the biomarker is a molecular biomarker.

The method comprises an image pre-processing step S201. The image pre-processing step S201 comprises obtaining a first set of image portions from an input image of tissue.

In an example scenario, a whole slide image (WSI) scanner scans an entire tissue slice. The whole side image, comprising around 60 000 pixels height by 60 000 pixels width, is then split into contiguous portions, or tiles, in the initial processing step S201. The image portions have a fixed input height and width. The portions may be contiguous or overlapping within the image. For example, the image portion size may be 512 x 512 pixels. An input image is first split into portions of this dimension. Other portion sizes may of course be used. For example, a portion size corresponding to a power of 2 may be used, for example: 128 x 128, 256 x 256, 512 x 512, or 1024 x 1024 pixels. Each input image may be of a different size, and therefore a different number of portions may be extracted from the input image depending on the size of the input image.

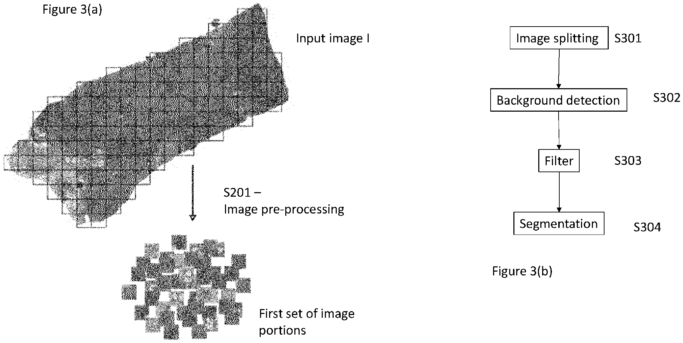

These image portions may form the first set. Alternatively, further steps may be performed in the image pre-processing stage S201 to eliminate tiles, such that the remaining tiles only form the first set, as will be described further in relation to Figure 3(a) below. For example, the image portions may be processed to eliminate any image portions that do not contain any cancer cells. Thus not all of the image portions from the original image are necessarily included in the first set.

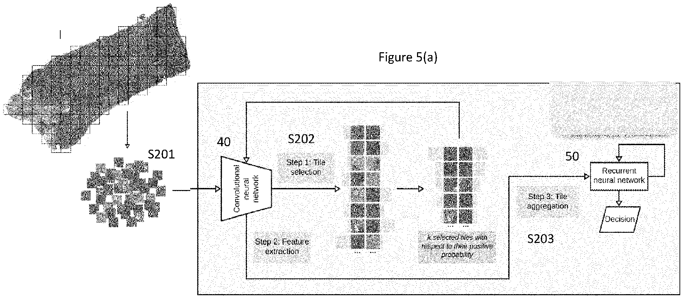

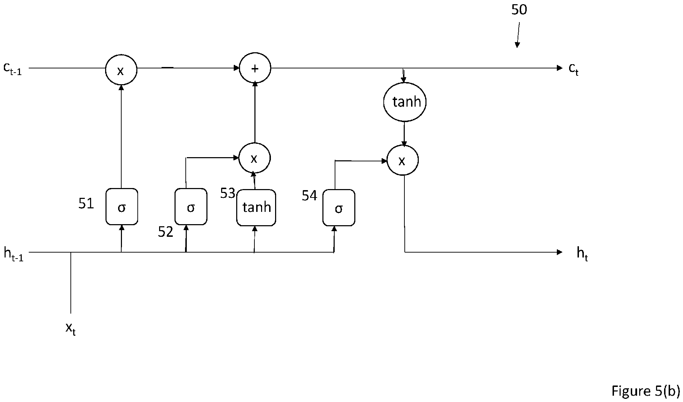

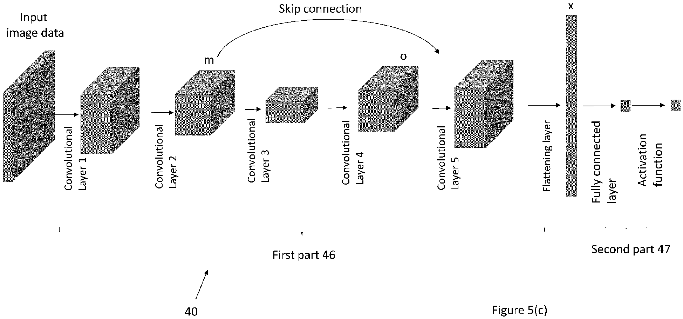

In S202, a step of selecting a second set of one or more image portions from the first set of image portions obtained in S201 is performed. In this stage, image data of each image portion in the first set is inputted into a first trained model comprising a first convolutional neural network. The first trained model generates an indication of whether the image portion is associated with a biomarker. This stage is described in more detail in relation to Figure 5 below. A reduced set of one or more image portions, the second set, which has fewer image portions that the first set, is obtained in the S202. The second set comprises one or more representative image portions, as determined form the output of the first trained model.

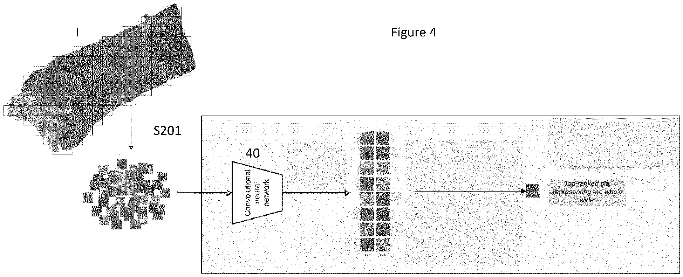

In S203, an indication of whether the input image is associated with the biomarker is determined from the second set of one or more image portions. In some embodiments, the indication is generated using a non-trainable function, for example a max pooling operator as described in relation to Figure 4. In other embodiments, first data corresponding to the second set of multiple image portions is input into a second trained model. Various examples of the second trained model are described below in relation to Figures 5 to 7.

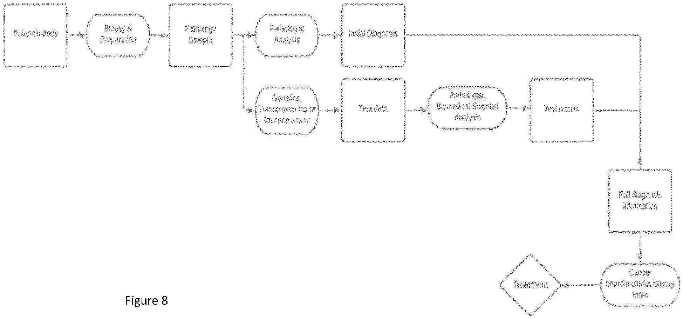

As described above, modern cancer diagnosis and treatment may rely on understanding the specific molecular profile of the cancer and patient in general. To that end, various medically relevant biomarkers may be tested through means of genetic, transcriptomics and immunological assays in order to determine how well a patient would respond to certain therapies. These tests are conducted on human biopsy samples. The testing takes, depending on the type of test and sample, anywhere between 1 and 30 days and is prone to procedural error. The results are then analysed by experts, which is again time-intensive and highly vulnerable to human error. Figure 8 shows a schematic illustration of such a cancer diagnosis pipeline.

Determining an indication of a specific biomarker automatically from an image of cancer tissue may shorten the time of such a process. Furthermore, reliability may be improved through removal of human errors. Such an automated system may help pathologists and others with their decision and improve the sensitivity of the process for example.

In order to make such a determination, a machine learning model may be trained using a training dataset. For example, a training dataset may comprise many whole slide images, each image being labelled as to whether or not the specific biomarker is present in the patient.

An input image may be processed in portions (tiles). By eliminating tiles which do not correspond to cancer tissue in a pre-processing step for example, the amount of data to be processed is reduced and reliability may be improved. This also improves interpretability of the results, since specific regions of the image corresponding to the biomarker may be identified. However, training a model to determine an indication of whether a portion of an input image of tissue is associated with a specific biomarker may be challenging. Such a problem is an example of a multi-instance learning (MIL) problem, where a label is associated with a whole slide image (WSI), rather than each individual instance (tile). This is different from a classification problem where one-to-one mapping is assumed to hold between an instance and a class. In a MIL setting, the data is weakly labelled, i.e. only one class label is provided for many instances, making the problem inherently more challenging. In order for an image to be labelled as positive, it must contain at least one tile of positive class, whereas all the tiles in a negative slide must be classified as negative. This formulation allows labels of individual instances to exist during training. However, their true value remains unknown. A means of aggregating tiles in order to obtain an image-level probability is therefore used.

The aggregation may be performed using a non-trainable function. Pooling operators, such as the maximum operator, can be used in an instance-level classification setting, which involves a classifier returning probabilities on a per-tile basis and aggregating individual scores through a max operator. An example of such a method is shown in Figure 4. In this method a second set of one image portion is selected from the first set of image portions using a classifier, and an indication of whether the input image is associated with the biomarker from the second set is determined from this image portion.

Such aggregation methods may provide unreliable image-level predictions in some cases due to the individual labels of tiles being unknown during training however. Furthermore, relying only on a single tile may not adequately represent an image in all cases. In particular, a WSI may contain hundreds of tiles with similar characteristics. In some embodiments, the output of the classifier is used to select a second set of multiple image portions, which are then used to represent the image. This makes the method applicable to any size of image, since regardless of the number of tiles in the image, only the second set, for example the top k tiles, are used to determine an indication of whether the input image is associated with the biomarker. A "max-pooling" based tile-selection may be used to acquire a representative set of tiles. An indication of whether the input image is associated with the biomarker is then determined by inputting the data from the representative set of tiles into a second trained model, which performs the aggregation. The aggregation operator comprises a neural network. Fully trainable aggregation operators, rather than pre-defined and non-trainable aggregation operations such as max-pooling, allow improved reliability. Parameterization of the aggregation increases the reliability. The method uses a learnable aggregation function and a tile selection procedure integrated to the model.

Furthermore, the full model, including the aggregation step, may be trained in an end-to-end manner, further improving reliability.

Determining the presence of a biomarker from image data is more challenging than, for example, tumour detection. However, by using a combination of image portion selection and aggregation, reliable classification may be obtained.

Various example methods will be described in relation to Figures 4 to 7, in which different aggregation operators are used. Figure 4 shows an example method in which a non-trained function is used as the aggregation operation, whereas Figures 5 to 7 show examples in which the aggregation operator includes a trained model. In the methods shown in Figures 4 to 7, a second set of one or more tiles is selected in S202 based on the output of a first CNN 40 classifier. This second set of tiles is then processed in S203 to generate an image level indication. However, the image pre-processing step S201 will first be described in more detail in relation to Figure 3(b).

Figure 3(b) shows a schematic illustration of an image pre-processing step S201 used in a method in accordance with an embodiment. Figure 3(a) shows a schematic illustration of the input image I, which is an image of a histological section stained with hematoxylin and eosin stain, and the output, which is a first set of image portions.

In S301, an input image, for example a WSI, is subdivided into fixed-sized portions, or tiles. In this example, each portion has an aspect ratio of 1:1, i.e. each portion is a square image. While tile generation can be performed at different magnification levels and with varying amounts of overlap between adjacent tiles, a simple tiling strategy may comprise acquiring patches of 512x512 pixels from the first slide level, with no overlap between tiles.

A background detection step is then performed, to eliminate any tile which is largely background. The background areas are the "white" areas as seen in the figure. Various image pre-processing techniques can also be utilised in the pre-processing step S201, including Gaussian filtering, histogram equalisation, colour normalisation, and image de-noising, allowing a better detection of foreground objects when the images suffer from artefacts or poor contrast.

In S302, a background detection algorithm is applied. The background detection is performed on a "thumbnail" of the image, i.e. a lower resolution copy of the entire image. The thumbnail is a lower-resolution snapshot of an image, e.g. a WSI. For example, the original image may be 60,000x60,000 pixels, whereas the thumbnail is 1024x1024 pixels for example. This step is used to segment the tissue from the background and the corresponding output mask is resized to match the resolution of the original image, in the manner described below.

In this step, the image is first converted to grayscale.

Background segmentation (or tissue extraction) starts with applying edge detection convolution kernels on the input image in order to locate pixels with high spatial frequency. A convolution between an edge detection kernel and the image is performed. The kernel is a small matrix of pre-defined values, for example:

$$\begin{bmatrix} -1 & -1 & -1 \\ -1 & 8 & -1 \\ -1 & -1 & -1 \end{bmatrix}$$

A plurality of edge detection kernels may be applied in this step for example a pair of 2x2 kernels in the form of [+1, 0; 0 -1] and [0, +1; -1, 0] may be used.

This step highlights regions where there exists a transition. The edge detection step outputs the gradients of the image. High gradients correspond to edges or transitions. Tissue regions generally contain many more transitions than background regions. As a result, tissue regions will be highlighted in this step.

The gradients are further smoothed with a Gaussian kernel. A convolution between a Gaussian blur kernel and the image is performed. The purpose of this step is to blur-out pixels, so the binarisation performed in the following step will have fewer artifacts. This essentially smooths the highlighted regions. The smoothed gradients highlight the foreground pixels.

The blurred image is binarized with a histogram-based thresholding method. This step replaces each pixel value with a value 1 if the pixel value is greater than some threshold T and a value 0 if the pixel value is less than the threshold. The threshold is determined for each tile using a histogram-based method such as Otsu's method, in which the threshold is determined by minimizing intra-class intensity variance, or equivalently, by maximizing inter-class variance (the classes being "background" and "foreground"). In order to reduce the computation required for this step whilst maintaining performance, the resolution (i.e. the number of histogram bins) can be selected based on a measure of the entropy, where images with higher entropy are processed with higher resolution. Alternative histogram based methods, such as triangle thresholding may be used.

A median filter is convolved over the binary mask to remove non-salient components.

Finally, holes in the foreground are filled to minimise the likelihood of acquiring false negatives within tissue. Various known algorithms may be used in this step, including A* and connected component analysis algorithms.

The tiles that are partially on the background, for example 80% of pixels are indicated as being background (pixel value is 0), are then removed from further analysis.

In S303, a standard deviation operation is used to eliminate any "all-white" tiles that may have survived the previous step. The standard deviation operation is applied to each image portion (tile) output from the previous step. In this step, the standard deviation of the pixel values output from the previous step is taken. A single value is returned, which is the standard deviation of all the pixel values within the tile. This value will be low if most of the pixels are "white". Tiles which output a value lower than a threshold value are eliminated in this step. A threshold value may be determined that provides good performance.

After S303, tiles that are largely foreground (i.e. tissue) remain, and are processed in the following steps.

In S304, a step of cancer cell segmentation is performed. The outcome of the cell segmentation step is used to eliminate tiles that do not contain any cancer cells, so that only image portions that are relevant for the task at hand are inputted to the subsequent steps. The tiles containing only non-cancer tissues are discarded.

A trained model can be used to perform cell segmentation. The model is configured to convert pixels into class labels, e.g. cancer cell and background. A segmentation model M trained to identify cancer tissue at a cellular level is used to eliminate tiles that do not contain any cancer cells. An example model M is described below. However, various methods of segmenting the tile image may be used. The original tile images are input to the model M (excluding those which have already been eliminated in S302 and S303).

The model M generates a value corresponding to each of a plurality of pixels representing whether the pixel corresponds to a cancer cell. Classification is performed for each pixel of the input image portion, to segment the image into two classes: regions of cancer tissue and regions which do not contain cancer tissue. The model M performs semantic image segmentation, meaning that each pixel in the input image is classified. The classification in this case is performed into two categories - the output of the model comprises two values indicating whether the pixel corresponds to cancer tissue or non-cancer tissue. The output has the same height and width as the input portion. For example, where the input data has a height of 512 pixels and a width of 512 pixels, the output is an array of values having height 512 and width 512. The values indicate the category.

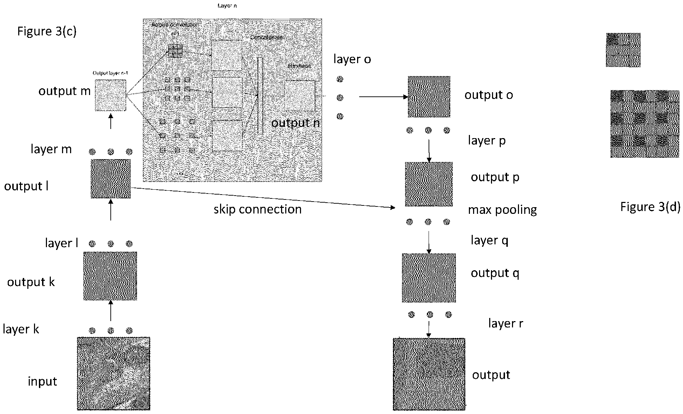

An example model M will now be described in relation to Figure 3(c), which shows a schematic illustration of an example segmentation model M based on a CNN. In the output, the different shaded regions of the output image correspond to the regions of cancer tissue and the regions which are not cancer tissue.

In practice many more layers are likely to be included, however the figure serves to illustrate how the spatial dimensions may be varied throughout the layers. The model M may comprise over 100 layers for example. In general, different types of layers and different numbers and combinations of layers are possible in order to implement the model M for various use cases.

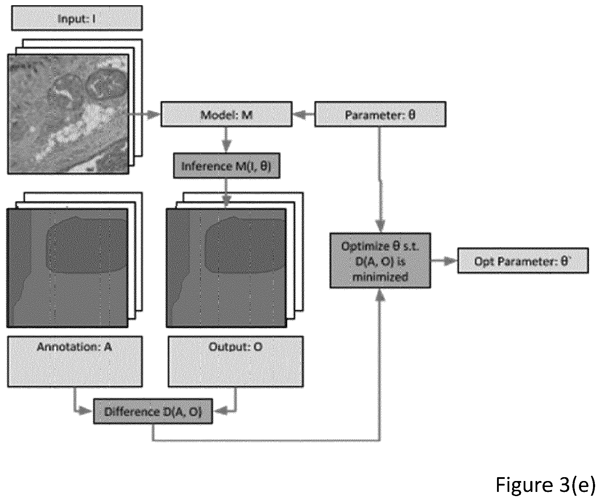

The model M comprises a convolutional neural network (CNN). A CNN is a neural network comprising at least one convolutional layer. The model M comprises a plurality of convolutional layers, with various filters and numbers of filters, generating output volumes of various sizes. The filter weights are trainable parameters which are updated during the training stage, described below in relation to Figure 3(e).

Pixel data can be directly input into a CNN. The first layer in the CNN is a convolutional layer. Each filter in the first layer has a depth matching the depth of the input data. For example, where the input data is RGB, the filter depth in the first layer is 3.

The output volume of the first layer is determined by a number of factors. The depth of the output volume of the layer corresponds to the number of filters. In an embodiment, there are 32 filters in the first layer, and therefore the output of the first layer has a depth of 32. The filters in the subsequent layer will therefore have a depth of 32. The height and width of the output volume is determined by the height and width of the input, the receptive field size of the filters (both height and width) and the filter stride. When the stride is 1 then the filters slide one pixel at a time. When the stride is 2 then the filters slide 2 pixels at a time, producing a smaller output volume. Any zero padding used at the borders will also affect the output size.

Each filter is moved along the width and height of the input, taking a dot product at each position. The output values for one filter form a 2D array. The output arrays from all the filters in the layer are stacked along the depth dimension, and the resulting volume input into the next layer.

The model M comprises a plurality of layers for which the output has a smaller dimension than the input. For example the height and/or width may be smaller than the input. In this manner, the height and width of the output may decrease through a number of the layers, whilst the depth increases. For example, there may be a first layer for which the output has a smaller height and/or width than the input, followed by one or more layers for which the output has the same dimension as the input, followed by a further layer for which the output has a smaller height and/or width than the input. For example, the first layer may take as input the image data (513 x 513 x 3) and output a volume (257 x 257 x 32). This layer applies a convolution using 32 filters, each of which outputs an array of volume 257 x 257. The height and width is reduced whereas the depth is increased. The height and width can be reduced by adjustment of the filter hyper-parameters (e.g. stride) for example. Since the output of the model M has the same height and width as the input, the model M also includes at least one layer for which the output has a larger dimension than the input. The model M may have an "encoder/decoder" structure, whereby the layers first decrease the height and width, whilst increasing the depth (via the filter hyper-parameters such as stride size for example) and then increase the height and width whilst decreasing the depth (via pooling layers and/or bilinear up-sampling layers for example).

The model also comprises one or more activation layers. For example, the model may comprise one or more RELU (rectified linear unit) layers, which apply an elementwise activation function. A batch normalisation layer may be implemented after each convolutional layer. An activation layer may be implemented after a batch normalisation layer. The model may comprise one or more units comprising a convolutional layer, a batch normalisation layer and an activation layer, or comprising a first convolutional layer, a first batch normalisation layer, a second convolutional layer, a second batch normalisation layer and an activation layer.

As well as one or more standard convolutional layers, the convolutional neural network further comprises a hidden layer comprising a dilated convolution. This layer may be referred to as an Atrous convolution layer. An Atrous convolution may also be referred to as a dilated convolution. A schematic illustration of a filter which performs a dilated convolution is shown in Figure 3(d). The dilated convolution shown in Figure 3(d) has a dilation factor of 2, and the filter has a receptive field size of 3 x 3. The dilated convolution operation (represented as *l) for a general unbounded case between an input I and a filter f with a dilation factor of l is:

$$(f *_l I)_t = \sum_{\tau=-\infty}^{\infty} f_\tau \cdot I_{t-l\tau}$$

The dilated convolution used in the convolutional neural network layer is bounded by the input size. Where the dilation factor is 1, the operation is the standard convolution operation as described above. Where the dilation factor is 2, as illustrated in Figure 3(d), at each position the dot product of the filter values with input values spaced one apart is taken. The filter is moved along the width and height of the input according to the stride in the same way as before. However, the entries from the input are spaced apart by a distance determined by the dilation factor. Increasing the dilation factor thus broadens the effective receptive field for the filter without increasing the filter size, i.e. without increasing the number of parameters. Having a dilation factor of > 1 means that non-local features can be learned, without increasing the number of parameters. Including a dilated convolution operation delivers a wider field of view without an increase in the number of parameters, and therefore computational cost. The receptive field can effectively be expanded without loss of resolution. Atrous convolution can also be defined as convolution of gapped sampling. By including convolutions with different dilation factors, both local and non-local features can be learned.

In the example shown, there is a single layer n comprising dilated convolutions. The layer comprising the dilated convolutions is located prior to the pooling and upsampling layers. The location of the layer comprising the dilated convolutions can be selected to be at various stages of the network depending on the use case. For example, by locating the layer comprising the dilated convolutions further through the network, higher level features can be learned in this layer.

In the nth layer of the model M, multiple separate convolution operations are performed in parallel on the data taken as input to the layer. Each convolution operation is performed as a separate filter. At least one of the convolution operations is a dilated convolution. One or more of the filters may have different dilation factors. In the layer n shown, two of the convolution operations shown are dilated convolutions, having different dilation factors. The first convolution is a standard convolution having a first dilation factor being equal to 1, the second convolution is a dilated convolution having a second dilation factor being equal to 2, and the third convolution is a dilated convolution having a third dilation factor being equal to 3. However, various combinations may be implemented, and various numbers of filters may be included.

Each filter takes the same input (i.e. being the output data from the previous n-1 layer). Each filter therefore has the same depth as the output from the n-1 layer. Each filter has a different dilation factor. The layer may comprise a combination of Atrous convolutions with various dilation factors. The filters perform their operations in parallel, in the same manner as the filters in the standard convolution layers. Each filter outputs an array of values. The arrays may be of differing sizes. The values from the output arrays are concatenated into a vector, which is then re-shaped to form a 2D array. This array is taken as input to the n+1 layer. The output of the filters is therefore combined and input into the subsequent layer.

Different convolution operations having different dilation factors are implemented in a single layer. By doing this, the layer is able to learn correlation of both local and non-local information at the same time, therefore allowing the learning of higher order spatial context. Information about both local and non-local features is propagated through the network. This is helpful for learning tissue morphology for example.

The layer n may comprise four filters, having dilation factors 1, 4, 8 and 12. However, various combinations of filters are possible. Although in the figure, the output of each filter is shown as having the same dimension, in practice each filter may have different output dimensions. The dilated filters may have a stride of 1. The dilated filters may have the same receptive field size. The receptive field size may be the same as the previous layer.

The model further comprises a skip connection. In practice, the model may comprise multiple skip connections, however for simplicity a small number of layers and a single skip connection is shown. A first layer m generates an output, referred to as output m, having a dimension smaller than the output of a previous layer. In this case, the output m is smaller than the output I and also smaller than the output k. Thus the output m is smaller than the output of the immediately previous layer I and is also smaller than the output of previous layer k.

A second layer q is subsequent to the first layer m. The input to the second layer q is generated from the input of the first layer m (also referred to as output I) as well as the output of the layer immediately prior to the second layer q (i.e. the output of the layer p). Inputting the output from the earlier layer directly to the later layer may be referred to as a "skip connection". The input of the first layer m is combined by pixel-wise addition with the output of the layer p. The result is then input into the second layer q. The skip connection may be implemented by including a pixel wise addition layer which combines the inputs. If the skip connection is implemented by pixel-wise addition, the inputs must have the same dimension. In this case, the skip connection is implemented between layers having the same dimensions. For example, the first and second layer are selected such that the input of the first layer m is the same dimension as the output of the layer p (immediately prior to the second layer).

Using one or more skip connections, information from the downstream is fed directly to the upstream. This maintains high level global and regional visual features throughout the network. These are useful for large patch segmentation. Including the skip connections may be referred to as a "ladder" approach. In one or more of the layers, the output is smaller than the input. Inputting features from an earlier layer directly into a later layer, skipping one or more intervening layers, provides context.

As well as convolutional layers, the model comprises one or more pooling layers. For example, pooling layers may be included to vary the spatial size. The pooling layers may be used to increase the width and/or height and decrease the depth of the output for example. The pooling layers may be "average pooling" layers. An average pooling layer comprises a filter having a spatial extent and stride, which is moved across the input, taking the average value at each position. Functions other than the average can be used however, for example, max pooling. Up-sampling layers, for example one or more bilinear up-sampling layers may additionally or alternatively be included in order to increase the height and/or width of the output layer.

The model may further comprise one or more pixel-wise addition layers and/or concatenation layers. These layers act to combine the outputs from two or more previous layers.

One or more fully connected layers may be included after the convolutional layers. A dropout layer may also be included to mitigate overfitting.

There is a single output for each category for each pixel. A further activation function is applied at the output, in a pixel-wise fashion, for example a binary softmax function. The activation function takes as input the values for the pixel, and outputs a probability value. Thus the final activation function outputs, for a single pixel, a probability value between 1 and 0 for each category. The final layer generates an output having the same height and width as the input. The depth of the output is equal to the number of categories, in this case 2 (whether the pixel corresponds to cancer tissue or non-cancer tissue). The output depth can be set by a convolutional layer having a number of filters corresponding to the desired output depth (i.e. desired number of categories). This convolutional layer may be located prior to the final layer, where the final layer is an up-sampling layer (for example using a transposed convolution) having the same output depth for example. The values in the output array indicate whether the pixel corresponds to that category or not, in this case whether pixel corresponds to a cancer cell for one category and whether the pixel corresponds to background for the other category.

A value greater than or equal to 0.5 for the cancer tissue category is then rounded to 1 (indicating cancer tissue). This threshold may be varied as a hyperparameter. A single matrix of values, with a value 1 (cancer tissue) or 0 (not cancer tissue) for each pixel is produced as the final output, for example by combining categories. The output shown in the figure indicates whether cancer tissue is present for the pixel or not.

Image portions (tiles) corresponding to outputs that do not contain any cancer cells, e.g. greater than 80% of output pixel values are 0 for the category cancer tissue, are then eliminated. A threshold between 75% and 80% may be selected. The threshold value may be varied as a hyperparameter, and a value which provides good performance determined. The original tiles corresponding to the remaining tiles form the first set of image portions, and are used in the subsequent steps S202 and S203. Since each image is of different size and contains a varying amount of cancer tissue, each input image may result in a different number of output tiles in the first set, ranging from a few dozens to a few thousands per input image.

Optionally, a colour normalisation process is applied to the tile images prior to inputting the image data to the subsequent steps S202 and S203. A challenge in automatic histopathological imaging systems is the variance across whole slide images with respect to their color distribution. This variation can be attributed to differences in staining and slide preparation procedures as well as the type of scanner and other hardware-related parameters. Diversity in color stands as an obstacle especially for pan-cancer studies, which may cover multiple datasets acquired at various sites. In addition, it may have a severe impact on the generalizability of a computational model to other datasets, which are likely to be very different from the dataset used to build the model in the first place. Generally when a model focuses on color features and associates them with the task at hand, it may fail on an unseen image acquired from a dataset in a different color spectrum. One option to deal with color variation is converting RGB images to grayscale. However, this may lead to loss of information which would otherwise be obtained from color channels.

An alternative to grayscale conversion is based on the method described in Ruifrok AC and Johnston DA: "Quantification of histochemical staining by color deconvolution".Analytical and quantitative cytology and histology 23: 291-299, September 2001. In this method, a process is performed to colour normalize a source tile to have the same "colour profile" as a target image. In an example described herein, histology images are stained with the Hematoxylin & Eosin (H&E) stains. These two chemicals typically stain: the nuclei a dark purple (Hematoxylin) and the cytoplasm a light pink (Eosin). Thus all pixels in an idealized histology image are principally composed of two colors. These stain colors vary from image to image and may be summarised in a stain matrix. A stain matrix M of both the source whole slide image and a target whole slide image are determined. The stain matrix M may be estimated using the method described in "A method for normalizing histology slides for quantitative analysis", Macenko et al, 2009 IEEE International Symposium on Biomedical Imaging: From Nano to Macro, 10.1109/ISBI.2009.5193250. The stain matrix is a matrix composed of two unit vectors: M = (h|e), where h and e are 3D vectors of colour of h stain and e stain:

$$M = \begin{array}{cc} H & E \\ \begin{bmatrix} \cdot & \cdot \\ \cdot & \cdot \\ \cdot & \cdot \end{bmatrix} & \begin{array}{c} R \\ G \\ B \end{array} \end{array}$$

Having estimated the stain matrices of the target and source, the colour normalised RGB pixel values for the source can then be determined. A given pixel stain density vector $c = \begin{pmatrix} c_H \\ c_E \end{pmatrix}$, has a pixel RGB optical density of $x = \begin{pmatrix} r \\ g \\ b \end{pmatrix} = Mc$. Equivalently, $c = M^{-1}x$.

Having estimated stain matrix $M_1$ of the source image and $M_2$ of the target image, to colour normalize pixel $x_1$ in source image to the target image colour profile, $c_1 = M_1^{-1}x_1$ is first determined. The inverted matrix $M^{-1}$ is determined using a projection onto its column space, such that $c_1$ is equivalently determined as $c_1 = (M_1^{T}M_1)^{-1}M_1^{T}x_1$. The colour normalised pixel is then calculated as $\hat{x}_1 = M_2c_1$.

Brightness normalisation may be applied, by taking the densities for each pixel in the source image (the c vector for each pixel) and shifting or re-scaling the values to match with the 99th percentile upper bounds for each stain density over pixels in the target image. The re-scaled pixel stain density vector c is then used to determine $\hat{x}_1$.

Figure 5(a) shows a schematic illustration of a method of processing an image of tissue according to an embodiment.

The method comprises a step of obtaining a first set of image portions from an input image of tissue S201, as has been described in relation to Figure 3 above. Each image portion identified in S201 is taken as input to S202 in turn. The original image data of the image portions may be taken as input, i.e. the original pixel values. Alternatively, as has been described above, some pre-processing may be performed on the original pixel values, for colour normalisation for example.

The image data for an image portion from the first set is inputted to a first Convolutional Neural Network (CNN) 40 in S202. This step is labelled "Step 1: Tile selection" in the figure. The first convolutional neural network 40 comprises a first part 46 comprising at least one convolutional layer and a second part 47, a classification part, which takes as input a one dimensional vector. The second part 47 may comprise at least one fully connected layer for example. The first CNN 40 is a multi-layer architecture of neural networks comprising a first part 46 comprising convolution filters applied to images at various layers of depth and field-of-view, followed by a second part 47 comprising fully connected dense layers and/or pooling layers for data reduction. The filter weights are trainable parameters which are learned during the training stage. While lower level filters detect coarse structures such as edges and blobs, deeper levels capture more complex properties like shape and texture and finally top layers learn to generalize on objects of interest with respect to the identification of the biomarker.

The first CNN 40 uses a binary classification. In other words, the CNN is used to determine whether the tile is associated with a specific molecular biomarker or not, i.e. a single class. Where it is desired to determine whether an image is associated with one of many possible biomarkers, a separate model may be used for each biomarker.

The tiles are submitted to the first CNN 40. Per-pixel data can be directly input into the first CNN 40. For each tile, the CNN outputs a probability the tile is assigned to the positive class (i.e. the tile is associated with the molecular biomarker).

The CNN may be based on a residual network architecture. A residual neural network comprises one or more skip connections. However, alternative architectures having capacity sufficient to capture the salient morphological features from the input images and correlate them with the target biomarker. Capacity may be determined by the network size and other architectural factors like number of layers, type of convolutions etc. An example CNN architecture based on a residual network architecture will now be described in relation to Figure 5(c), which shows a schematic illustration of an example first CNN 40. The figure shows a small number of layers for simplicity, however the first CNN 40 may comprise over 100 layers for example.

The first layer in the CNN is a convolutional layer, labelled "convolutional layer 1" in the figure. Each filter in the first layer has a depth matching the depth of the input data. For example, where the input data is RGB, the filter depth in the first layer is 3. For simplicity, the CNN shown in Figure 4(c) has an input data depth of 1 (i.e. grayscale input data).

The output volume of the first layer is determined by a number of factors. The depth of the output volume of the first layer corresponds to the number of filters. For example, there may be 32 filters in the first layer, and therefore the output of the first layer has a depth of 32. The filters in the subsequent layer will therefore have a depth of 32. The height and width of the output volume is determined by the height and width of the input, the receptive field size of the filters (both height and width) and the filter stride. When the stride is 1 then the filters slide one pixel at a time. When the stride is 2 then the filters slide 2 pixels at a time, producing a smaller output volume. Any zero padding used at the borders will also affect the output size. Each filter is moved along the width and height of the input, taking a dot product at each position. The output values for one filter form a 2D array. The output arrays from all the filters in the layer are stacked along the depth dimension, and the resulting volume input into the next layer.

Each convolutional layer may be followed by an activation layer. An activation layer applies an elementwise activation function, leaving the size unchanged. The activation layers are not shown in the figure for simplicity. For example, the model may comprise one or more ReLU (rectified linear unit) layers, which apply an elementwise activation function. A batch normalisation layer may be implemented after each convolutional layer. An activation layer may be implemented after the batch normalisation layer. The model may comprise units comprising a convolutional layer, a batch normalisation layer and an activation layer, or comprising a first convolutional layer, a first batch normalisation layer, a second convolutional layer, a second batch normalisation layer and an activation layer.

The first CNN 40 comprises a plurality of layers for which the output has a smaller dimension than the input. For example the height and/or width may be smaller than the input to the layer. In this manner, the height and width may decrease through a number of the layers, whilst the depth increases. The first CNN 40 may have an "encoder/decoder" structure, whereby the layers first decrease the height and width, whilst increasing the depth (via the filter hyperparameters such as stride size for example) and then increase the height and width whilst decreasing the depth (via pooling layers and/or bilinear up-sampling layers for example). This is illustrated in Figure 5(c), which illustrates the output sizes of the layers.

The model may further comprise one or more pooling layers. For example, pooling layers may be included to vary the spatial size. The pooling layers may be used to increase the width and/or height and decrease the depth of the output for example. The pooling layers may be "average pooling" layers. An average pooling layer comprises a filter having a spatial extent and stride, which is moved across the input, taking the average value at each position. Functions other than the average can be used however, for example, max pooling. Up-sampling layers, for example one or more bilinear up-sampling layers may additionally or alternatively be included in order to increase the height and/or width.

The model further comprises at least one skip connection. In practice, the model may comprise multiple skip connections, however for simplicity a small number of layers and a single skip connection is shown in Figure 5(c). The second layer "Convolutional layer 2" generates an output, referred to as output m. The fourth layer "Convolutional layer 4" generates an output o, having the same dimension as the output m. The input to the "Convolutional layer 5" is generated from the output of the first layer m as well as the output of the fourth layer o. Inputting the output from the earlier layer directly to the later layer is a "skip connection". The outputs in this example are combined by pixel-wise addition. Concatenation could alternatively be used, where the outputs are different sizes for example. Using one or more skip connections, information from the downstream is fed directly to the upstream. This maintains high level global and regional visual features throughout the network. Inputting features from an earlier layer directly into a later layer, skipping one or more intervening layers, provides context.

A flattening layer is included after the final convolutional layer. The flattening layer converts the output data from the final convolutional layer into a 1-dimensional vector x for inputting into the next layer. The layers prior to the flattening layer in this example form the first part of the CNN 46.

One or more fully connected layers are included after the flattening layer. The final fully connected layer outputs one value, corresponding to the positive class. An activation function is applied at the output, for example a sigmoid, to give a probability value. The activation function takes as input the value output from the final fully connected layer and normalizes to a probability. Thus the activation function outputs a value between 1 and 0. The fully connected layer(s) and the activation function form the second part 47 of the first CNN 40.

For each tile, the CNN outputs a probability the tile is assigned to the positive class (i.e. the tile is associated with the molecular biomarker). The tiles are then ranked according to their probability of being assigned to the positive class. A second set of two or more image portions (tiles) are then selected. This may comprise selecting the tiles corresponding to the top k probabilities for example, where k is an integer greater than or equal to 2. The second set of tiles corresponds to the top k tiles, i.e. the k tiles having the highest probabilities. These tiles are selected to represent the image in the remaining steps. In an example, k=100. However, k may be determined as a hyper-parameter. The value may be lower or higher for example.

In S203, an indication of whether the input image is associated with the biomarker is determined from the second set of image portions. S203 comprises two stages. The first stage is "Step 2: Feature extraction". In this step, first data corresponding to each tile in the second set is generated. The second stage is "Step 3: Tile aggregation". In this step, the first data corresponding to the second set of image portions is inputted into an aggregation module. In this example the aggregation module comprises a trained recurrent neural network (RNN) 50.

The first data is extracted using the first convolutional neural network 40, omitting the classifier layer, i.e. omitting the second part 47. The tiles in the second set are processed in order to extract a set of features corresponding to each image portion (tile). In particular, a d-dimensional feature vector x is generated corresponding to each of the top k tiles (the second set of tiles). For example, the d-dimensional feature vector x may be the output of the flattening layer, as shown in Figure 5(c). The feature vector x is generated by inputting the image data for each image portion (tile) of the second set again into the first CNN 40, omitting the final classifier layer of the first CNN 40. The CNN may be used as a feature extractor, since it can capture tissue properties within tiles throughout a set of convolutional filters applied to images at various layers of depth, effectively encoding the high-level visual features into a low dimensional embedding. Once the linear classifier layer is removed, the pre-trained first CNN 40 is used to transform the representative tiles into an embedding of d-dimensional feature vectors, in which d depends on the architecture of the CNN. These vectors may be seen as the "fingerprints" of the representative tiles.

The top k tiles are selected in S202 and processed in S203. The top k tiles, i.e. the k tiles having the highest probabilities, are selected to represent the image in the remaining steps. In S203, the top k tile images are first processed in order to extract a set of features corresponding to each image portion (tile). In particular, a d-dimensional feature vector x is generated corresponding to each of the top k tiles (the second set of tiles). The value of d depends on the output size of the flattened layer, so changes depending on the architecture. For example, d may be 512. The input to S203 thus comprises a set of k image portions (tiles), which were selected based on the output of the first CNN 40. The k image portions are then fed through the first CNN 40 again, omitting the classification layer, to generate a d-dimensional feature vector x corresponding to each of the k tiles. This results in a sequence of k d-dimensional feature vectors. Each d-dimensional feature vector corresponds to an image portion (tile). The k feature vectors correspond to the k tiles output from the CNN 40 in the tile selection step S202. The sequence of feature vectors is ordered with respect to the probabilities output from the first CNN 40 in step S202.

This sequence of feature vectors is then submitted to a recurrent neural network (RNN) 50, to achieve the final image-level determination as to whether the image is associated with the biomarker. In this step, an indication of whether the input image is associated with the biomarker is determined by combining or aggregating the data, in this case the feature vectors, corresponding to the second set of one or more image portions using the RNN 50. The recurrent neural network 50 is a fully trainable aggregation operator based on neural networks.

Using an RNN allows integration of the information at the representation level into the slide-level class probability by modelling the sequential dependency across tiles through a set of hidden layers. Furthermore, it has the potential to fix errors made during tile selection in the steps prior to the RNN module 50, which in the case of max pooling, could be incorporated into the final model output and potentially affect the performance. For example, for an image which is not associated with the specific biomarker, one tile may result in an erroneously high probability. If the result for the entire image is taken from only this tile, an erroneous result will be returned. However, the RNN will take into account the other k-1 tiles.

Different recurrent neural networks may be used, such as those with ReLU and tanh activation functions, as well as more sophisticated modules including gated recurrent unit (GRU) and long-short term memory (LSTM). In cases where the number of tiles k is set relatively high (e.g. k is of the order of 50 to 100), an LSTM may be seen to perform better. Networks using ReLU or tanh may perform better with fewer tiles.

An example RNN 50 based on an LSTM structure will be described here. An LSTM structure provides resistance to "forgetting" the early instances in the sequence. Figure 5(b) shows an example RNN 50 based on an LSTM structure, which may be used in the method described in relation to Figure 5(a). As is described below, the LSTM comprises a plurality of neural network layers.

The d-dimensional feature vectors output from the first CNN 40 in the feature extraction step are labelled in this figure as $x_t$. As explained above, there are k feature vectors, such that t runs from 1 to k. Thus the feature vector corresponding to the least probable tile is $x_t$, and the feature vector corresponding to the most probable of the k tiles is $x_1$. The tiles are submitted in decreasing order of probability - the first tile that is inputted to the RNN is the one with the highest probability. Each feature vector of length d is inputted in to the LSTM 50 in sequence, with $x_1$ input first, and $x_k$ input last. At each step in the sequence, the LSTM 50 outputs a vector ht corresponding to each input vector $x_t$. The size of $h_t$ is a hyper-parameter, and may be 128 or 256 for example. The output $h_k$ of the final step in the sequence is used to generate an indication of whether the input image is associated with the biomarker. The number of steps is equal to the number of selected tiles k.

The $\sigma$ and tanh in the boxes each represent a learned neural network layer with the respective non-linear activation function indicated (sigmoid and tanh). The dimension of the layers is a hyper parameter - 128 or 256 may be used for example. The tanh, addition and other operations in the circles represent point-wise operations. The output $h_t$

for the input feature vector $x_t$ is passed on to the next time step, and input at the point indicated by $h_{t-1}$. Furthermore, the output cell state $c_t$ is passed on to the next time step and input at the point indicated by $c_{t-1}$.

The input feature vector $x_t$ and the output from the previous time step $h_{t-1}$ are concatenated, to form a single combined vector, referred to here as the first combined vector. The LSTM then comprises four neural network layers, 51, 52, 53 and 54, three having a sigmoid activation function and one having a tanh activation function.

The first sigmoid layer 51 takes the first combined vector as input, and outputs a second vector comprising values between 0 and 1. The second vector has the same length as the cell state C, such that each value corresponds to an entry in the cell state. The cell state from the previous step $C_{t-1}$ is multiplied with the second vector in a pointwise multiplication (Hadamard product) to give a third vector, again having the same length as the cell state. The second vector essentially determines what information is kept from the previous cell state $C_{t-1}$. Cell state C is a vector of length hidden size H, e.g. 128 or 256. All the variables such as cell state C and ht are vectors of length H.

The second sigmoid layer 52 again takes the first combined vector as input, and outputs a fourth vector comprising values between 0 and 1. The fourth vector again has the same length as the cell state C, such that each value corresponds to an entry in the cell state.

The tanh layer 53 again takes the first combined vector as input, and outputs a fifth vector comprising values between -1 and 1. The fifth vector again has the same length as the cell state C, such that each value corresponds to an entry in the cell state.

The fourth vector is multiplied with the fifth vector in a pointwise multiplication (Hadamard product) to give a sixth vector, again having the same length as the cell state. The third vector and sixth vector are then added in a pointwise vector addition to give the cell state for the current time step, Ct.

The third sigmoid layer 54 again takes the first combined vector as input, and outputs a seventh vector comprising values between 0 and 1. The seventh vector again has the same length as the cell state C. The cell state values are each input to a tanh function, such that the values are set between -1 and 1. The output of this function is then multiplied in a point wise multiplication with the seventh vector, to give the output.

The output of each step is fed as the input to the next step. The weights and biases of each of the four neural network layers, 51, 52, 53 and 54 are learned before operation during the training stage, which will be described below. These are the trainable parameters of the LSTM. The output $h_k$ of the final step in the sequence is used to generate an indication of whether the input image is associated with the biomarker. The output $h_k$ of the final step in the sequence is inputted to a final fully connected layer, which results in two output values. A softmax function is then applied. This final step performs the classification. The input of the dense layer is the hidden size H, and the output size is 2. This final layer applies a linear transformation to the incoming data. A binary softmax is then applied. The value output for the positive class corresponds to a probability that the input image is associated with the biomarker.

Optionally, the feature vectors, or embeddings, are processed through the LSTM in batches, for example 10 at a time. In this case, the feature vectors in the batch are combined to form a matrix, and at each time step a matrix is inputted. The neural network layers are matrix neural network layers, and cell state C can be a matrix. Where the batch size B > 1 the cell state is a matrix of size B x H and the output ht becomes a matrix of B x H. The final classification layer in this case will also be a matrix neural network layer.

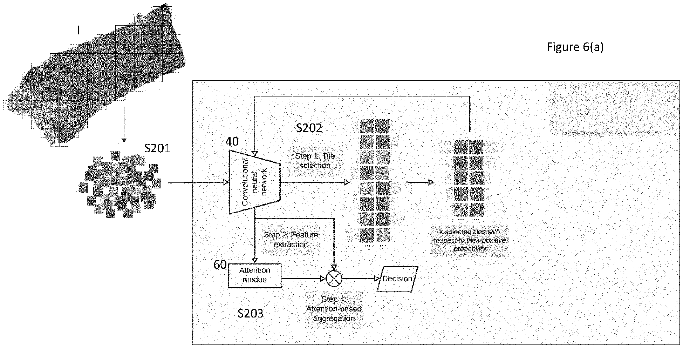

Figure 6(a) shows a schematic illustration of a method in accordance with an alternative embodiment. In this method, S201 and S202 are performed as described previously. The first CCN 40 "Step 1: Tile selection" outputs a probability for each tile that the tile is associated with the specific biomarker. The k tiles having the highest probabilities are selected and input into S203. These tiles are then inputted into the first CNN 40 again, in "Step 2: feature extraction", with the classifier layer omitted. The resulting d-dimensional feature vectors x, or embeddings, are combined into a kxd matrix, which is inputted to the attention module 60.

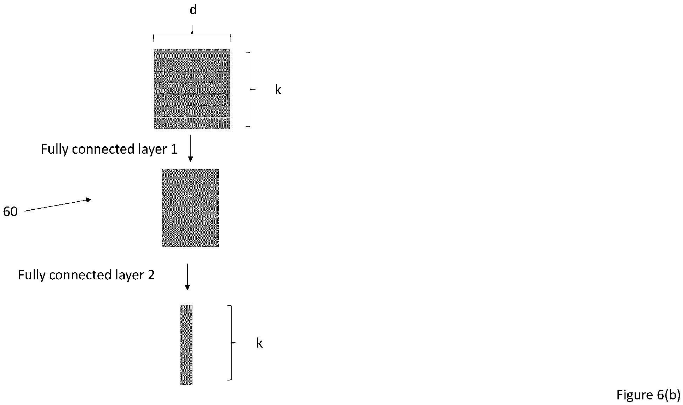

The attention module 60 is a fully-connected feed-forward matrix neural network that takes a kxd matrix as input. The output of the attention module 60 neural network is a k-dimensional vector. The attention module 60 therefore returns a weight vector, with each weight value corresponding to the contribution of a tile to the final model probability. The weight vector highlights the most important tiles for the prediction of molecular biomarkers. An example of an attention module 60 structure is shown in Figure 6(b). The first layer comprises a matrix of weights. The input kxd matrix is fed through the first layer, and an activation function applied (tanh or ReLU). The output is a kxg matrix, where the dimension g is the output dimension of the first layer. The value of g is a hyper-parameter - it may be 128 or 256 for example. The kxg matrix is fed into the second layer, which is also a fully connected layer. An activation function is applied. The output is a vector of length k, where each value corresponds to the weight. Although an example is described here, various other attention mechanisms could alternatively be used. For example, additional neural network layers may be included. For example, a gated attention module may be used.

The attention module 60 outputs a k-dimensional weight vector.

Each d-dimensional feature vector output from the first CNN 40 in the feature extraction step is multiplied by the corresponding attention weight, i.e. each value in the feature vector is multiplied by the weight. The weighted feature vectors are then combined into a matrix and passed to a classifier layer. This is a further fully-connected feed-forward matrix neural network layer. A sigmoid function activation function is applied. The output of the classifier layer is a single value of probability between 0 and 1. This is an indication of whether the input image is associated with the biomarker. The attention mechanism 60 is a fully trainable aggregation operator based on neural networks. The attention mechanism provides an alternative aggregation method to the recurrent neural network. The attention mechanism 60 allows the most important tile to be determined.

By weighting feature vectors with respect to their importance, not all tiles are taken into account equally for aggregation. Furthermore, the attention mechanism provides benefits in terms of interpretability, since the key tiles which trigger the classification are known.

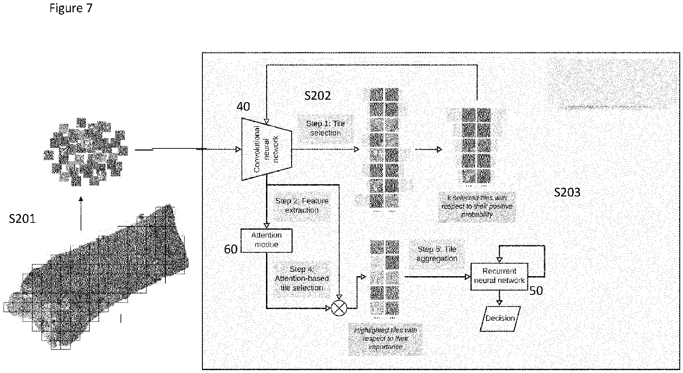

Figure 7 shows a schematic illustration of a method of determining an indication of whether the input image is associated with the biomarker used in a method in accordance with an alternative embodiment. The method uses an attention mechanism 60 together with an RNN 50 as part of the aggregation operator.

In this method, steps S201 and S202 are performed in the same manner as in the method of Figure 5(a). The top k tiles are selected in S202 and processed in S203. The top k tiles, i.e. the k tiles having the highest probabilities, are selected to represent the image in the remaining steps. In S203, the top k tile images are first processed in order to extract a set of features corresponding to each image portion (tile). This is done in the same manner as has been described above in relation to Figure 5(a). This results in a sequence of k d-dimensional feature vectors x. Each d-dimensional feature vector x corresponds to an image portion (tile). The k feature vectors correspond to the k tiles output from the CNN 40 in the tile selection step S202. The k feature vectors are combined into a kxd matrix, which is inputted to the attention module 60 in the same manner described in relation to Figure 6 above. The attention module 60 has been described in relation to Figure 6 above.

As explained above, by weighting feature vectors with respect to their importance, not all tiles are taken into account equally for aggregation. Furthermore, the attention mechanism provides benefits in terms of interpretability, since the key tiles which trigger the classification are known.

The attention module 60 outputs a vector of length k, as described above. This can be combined with the input to the RNN 50 in various ways.

In a first example, each d-dimensional feature vector output from the first CNN 40 in the feature extraction step is multiplied by the corresponding attention weight, i.e. each value in the feature vector is multiplied by the weight. The sequence of weighted feature vectors is then ordered with respect to the probabilities output from the first CNN 40. A trainable weighted average is therefore provided. In this step, each feature vector output from the first CNN 40 in the second pass is multiplied by its corresponding weight value. These weighted feature vectors are ordered with respect to the probabilities output from the first CNN 40 in the first pass. This sequence of weighted feature vectors is then submitted to the recurrent neural network (RNN) 50, in the same manner as described above, with the vector corresponding to the most probable tile input first.

In a second example, additionally or alternatively, the d-dimensional feature vectors are ordered with respect to the weight values output from the attention module 60. The d-dimensional feature vectors are then input to the recurrent neural network (RNN) 50, in the same manner as described above, with the vector corresponding to the most important tile input first.

In a third example, additionally or alternatively, and as shown in Figure 6, a step of further eliminating tiles from the analysis may be performed. The attention module 60 can be used to further decrease the number of tiles via ordering the feature vectors by attention weight and only passing the top n tiles to the final RNN module 50. In this case, step S203 comprises "Step 2: Feature extraction" as described above. The d-dimensional feature vectors x are then inputted to the attention module 60 as described previously. A further step, "Step 4: attention based tile selection" is then performed. The feature vectors are ordered with respect to the weights. A third set of image portions is then selected, corresponding to the top n feature vectors, where n is an integer greater than 1. The feature vectors corresponding to the third set of image portions is then submitted to the recurrent neural network (RNN) 50. The attention mechanism is used for ranking the most representative tiles and the RNN for aggregating them to achieve the image-level prediction. By eliminating tiles based on the output of the attention model 60, the computational intensive RNN step may be made more efficient, since fewer tiles are processed whilst maintaining reliability.

In the first and third example, the feature vectors may be input to the RNN 50 in order of importance or probability. In the second and third example, the original feature vectors or the weighted feature vectors may be submitted to the RNN 50.

The three methods described all use an attention-based aggregation module for combining tile-level information into image-level predictions. The attention module 60 provides a permutation-invariant means of aggregation for multi instance learning. A max-pooling based tile-selection step is used in S202 to acquire a representative set of tiles for the attention module. The method is therefore applicable to any size of image. An attention module 60 and recurrent neural network 50 are combined in this example in the aggregation module. In this example, the attention module 60 has a single attention branch.

In the above figures, aggregation modules comprising an RNN, attention module, or combination of the two are described. However, other trainable aggregation operators may additionally or alternatively be included in the aggregation module.

Alternatively, a non-trainable aggregation module may be used. Figure 4 shows a schematic illustration of an alternative method of processing an image of tissue according to an embodiment, in which a pooling operator is used. The method comprises a step S201 of obtaining a first set of image portions from an input image of tissue, as has been described above. Each image portion obtained in S201 is then taken as input to a first convolutional neural network 40, one at a time, in the manner described previously. The convolutional neural network 40 generates an indication of whether the image portion is associated with the biomarker. Thus the first CNN 40 is used to classify whether or not the tile is associated with a specific molecular biomarker for example, as described previously. For each tile, the CNN 40 outputs a probability the tile is assigned to the positive class (i.e. the tile is associated with the molecular biomarker). The tiles are then ranked according to their probability of being assigned to the positive class.

In this method, the top-ranked tile for the image is used to determine whether the molecular biomarker is present. Thus a second set of one image portion is selected from the first set of image portions output from S201 by inputting image data of each image portion into the first CNN 40. For example, it may be determined if the probability for the top ranked tile is greater than a threshold. The threshold may be 0.5 for example. The threshold may be a hyperparameter which is optimised to increase the performance. This is equivalent to max pooling. A pooling operator, such as the maximum operator in this case, is used. The first CNN classifier 40 returns probabilities on a per-tile basis, and these individual scores are aggregated through a max operator. Pooling operators such as the maximum operator can be suitable in an instance-level classification setting, which may involve a classifier returning probabilities on a per-tile basis and aggregating individual scores through a max operator. Other non-trainable aggregation functions, such as averaging, may be used.

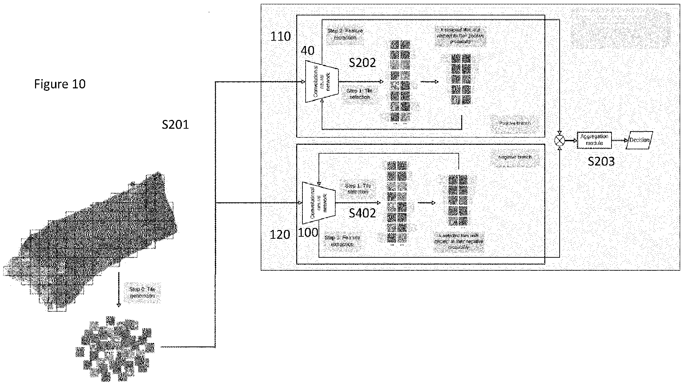

Figure 10 shows a schematic illustration of a method in accordance with an alternative embodiment. In this method, step S201 is performed as has been described previously. The image portions (tiles) are then processed in S202 and feature vectors are extracted in S203 as has been described previously. This is referred to as the positive branch 110.

A second series of steps, performed in parallel with S202 and S203, is also performed on the output of S201. These steps are referred to as the negative branch 120. In S402, a step of selecting a fourth set of one or more image portions from the first set of image portions obtained in S201 is performed. In this stage, image data of each image portion in the first set is inputted into a second convolutional neural network 100. The second CNN 100 may have the same structure as the first CNN 40. The second CNN 100 generates an indication of whether the image portion is not associated with the biomarker. In other words, the second CNN 100 generates a probability that the image portion is not associated with the specific biomarker. A reduced set of one or more image portions, the fourth set, which has fewer image portions that the first set, is obtained in S402 based on the output of the second CNN 100.

The fourth set of k image portions is then re-submitted to the second CNN 100, omitting the second portion, i.e. the classification layer, in order to extract a d-dimensional feature vector corresponding to each image portion.

The feature vectors are inputted to an aggregation module, which may comprise a trained aggregation operator such as an RNN, attention module, or combination of the two for example, as described in relation to Figures 5 to 7 above. The aggregation module outputs a probability that the image corresponds to the specific biomarker, again as described above.

The methods described in relation to Figures 5 to 7 only consider the positive class probabilities during inference, and assume that the model will learn to differentiate the negative class inherently. This may increase a model's tendency towards predicting a positive class more often than a negative. In order to directly incorporate the information from the negative class into the prediction capacity of the network, a dual-branch architecture may be used, as described in relation to Figure 10. Each branch is responsible for a specific class, i.e. the positive branch 110 accounts for the positive class probabilities whereas the negative branch 120 focuses on the negative class. Each branch can be realized with one of the neural network models described in the previous sections.

In the above described methods, various trained models were used. Example methods of training the various models will now be described.

Various methods of training the first convolutional neural network 40, and where relevant the aggregation module (comprising the RNN 50 and/or attention module 60 for example) as described above will first be described. A training data set comprising a plurality of images is used. The images may correspond to the intended type of input images for the model. In the example described above, the input images are images of a histological section stained with hematoxylin and eosin stain. Thus a training dataset of images of a histological section stained with hematoxylin and eosin stain may be used to train the models.

Each image is labelled depending on whether or not it corresponds to the specific biomarker that the model is to detect. As described above, the specific biomarker may be the ER biomarker, the HER2 biomarker, the PR biomarker, the EGFR biomarker or the MSI biomarker for example. The method may be used to detect various other biomarkers. If the model is to be used to determine an indication of whether the input image is associated with the ER biomarker for example, each image in the training data set is labelled with a 1 if it corresponds to the ER biomarker and 0 if it does not. In order to generate the labels, information from an IHC staining process may be used for example. For some datasets, an expert may review IHC-stained images and determine the ER/PR statuses of target images if they are not already available as metadata for example. These are then used as ground-truth labels for the H&E images to train the models. Various testing of human samples from the patient through means of genetic, transcriptomics and/or immunological assays may be used. These tests are conducted on human samples called biopsies, in liquid and/or solid forms, which then undergo the procedure to inform the molecular status of the sample. The results are then analysed by experts - pathologist for tissue biopsy, hematologist for liquid biopsy, cytopathologist for cytology samples, geneticist for genetic/transcriptomic assay etc - to generate a label 1 or 0. The annotation may be performed by a trained pathologist.

A training process comprising two stages will now be described, using the training data set.

In the first stage, during the training process, for each image in the training dataset, the same image preprocessing step S201 as described in relation to Figure 3(a) is performed. Thus for each image, a plurality of image portions are obtained, in the same manner as has been described above in relation to inference. As described above, cell segmentation may be used to discard the tiles containing only non-cancer tissues from the training dataset. In this case, the quality of the dataset used for training the model directly relies on the accuracy of the segmentation approach. A pre-trained model may be used for the cell segmentation.

The tiles are then paired with the labels of their corresponding slides and used to train the first CNN 40. Tiles are submitted to the first CNN 40 which generates a probability of being assigned to the positive class in the same manner as during inference.

The first CNN 40 has an associated parameter vector θ1. The parameters include the filter weights for all of the convolutional layers in the first part of the first CNN 40 as well as the weights for the second part of the first CNN 40. The goal of the training process is to find a parameter vector θ1' so that the difference between the annotations and the outputs is minimised.

The optimal parameters are computed by assigning random values as θ1 and then updating θ1 sequentially by computing the gradient of the loss $\frac{\partial D1}{\partial \theta 1}$ and updating θ1 using the computed gradient. D1 represents a loss function, which in this step is a "per-tile" loss. A binary cross entropy loss may be used. The gradient of the loss with respect to each of the trainable parameters of the model is determined through back-propagation. The gradients are then used to determine the updated parameters, using an optimiser function. This family of update methods is known as gradient descent (GD), generally defined iteratively as:

$$\theta 1' = \theta 1 - \mu 1 \frac{\partial D1}{\partial \theta 1}$$

where μ1 is the learning rate defining how quickly the parameters are updated. The update may be performed based on a batch average. A batch size of 8 tiles or 16 tiles is used for example.

An Adam optimization algorithm may be used. The optimisation strategy selected may depend on the performance of each strategy on a use-case however. For example, one of the following optimisation methods may be selected:

- Stochastic Gradient Descent (SGD)
- AdaDelta
- Adam
- AdaMax
- Nesterov Adam Optimiser
- RMSProp

Where the aggregation operation is a non-trained function, for example a max-ppoling step as described in relation to Figure 4, no further training is performed. However, where the aggregation operation is a trainable model, a second training stage is performed.

In the second training stage, the remaining tiles are then inputted into the first part of the first CNN 40, and a feature vector extracted for each tile in the same manner as during inference. The feature vectors are inputted to the aggregation module, comprising the RNN and/or the attention mechanism for example, and a final output value corresponding to the whole image is outputted.

The first part of the first CNN 40 together with the aggregation module (comprising the RNN and/or the attention mechanism) has an associated parameter vector θ2. The parameters include the filter weights for all of the convolutional layers in the first part of the first CNN 40, together with the weights of the RNN and/or the attention mechanism networks for example. The training process then finds a parameter vector θ2' so that the difference between the labels and the outputs is minimised. Here, labels corresponding to the whole slide are used.

The optimal parameters are computed by assigning random values as θ2 and then updating θ2 sequentially by computing the gradient of the loss $\frac{\partial D2}{\partial \theta 2}$ and updating θ2 using the computed gradient. D2 represents a loss function, which in this step is a "per-image" loss. A binary cross entropy loss may be used. The gradient of the loss with respect to each of the trainable parameters of the model is determined through back-propagation. The gradients are then used to determine the updated parameters, using an optimiser function. This family of update methods is known as gradient descent (GD), generally defined iteratively as:

$$\theta 2' = \theta 2 - \mu 2 \frac{\partial D2}{\partial \theta 2}$$

where μ2 is the learning rate defining how quickly the parameters are updated. The update may be performed based on a batch average. A batch size of 8 images or 16 images is used for example.

Again, an Adam optimization algorithm may be used. The optimisation strategy selected may depend on the performance of each strategy on a use-case however. For example, one of the following optimisation methods may be selected:

- Stochastic Gradient Descent (SGD)
- AdaDelta
- Adam
- AdaMax
- Nesterov Adam Optimiser
- RMSProp

The first training stage may be performed using all of the images in the training data set, and then the second training stage performed. Alternatively, a batch of images may be used in the first training stage, and then the second training stage performed. The first training stage may then be repeated with a second batch of input images and so on.

In this manner, the models are trained in a weakly-supervised setting. The training uses multiple-instance learning (MIL). MIL is a type of supervised learning. In MIL, instead of training data comprising instances (in this case image portions) which are individually labelled, the training data comprises a set of labelled bags (in this case images), each containing many instances. If the image does not correspond to the specific biomarker, i.e. it is labelled 0, none of the image portions in the image correspond to the specific biomarker. However, the image will correspond to the biomarker if one image portion corresponds to the specific biomarker. Images which are labelled positive therefore have at least one image portion which is positive. However, it may also comprise many image portions which are negative.

Each tile is associated with a positive (1) or negative (0) label indicating whether the specific molecular biomarker is present. The label is inherited from the parent image however. Thus a tile may be labelled as positive when the parent image is associated with the specific molecular biomarker, but the tile itself is not (since the region of tissue within the tile does not contain the molecular biomarker for example).

A multi-instance learning (MIL) approach is thus used. A label associated with a whole slide image (for example) is assigned to a set of multiple instances, i.e. tiles forming the WSI. This is different from a classification problem where one-to-one mapping is assumed to hold between an input instance and a class. Since in a MIL setting the data is weakly labelled, only one class label is provided for many instances of the same category. This makes training of the model to identify whether individual instances (tiles) correspond to the class inherently more challenging. In order for an image to be labelled as positive, it must contain at least one tile of positive class, whereas all the tiles in a negative slide must be classified as negative. This formulation ensures that labels of individual instances exist during training. However, their true value still remains unknown.

A means of aggregating tiles is included in S203 in order to obtain an image-level output, e.g. a probability. A training process comprising two stages may be used, where per-tile training is performed in the first stage, and a per-image end to end training method is performed in the second stage. The method can be trained in an end to end manner, since once the tiles are selected in the first stage, a forward pass is performed again with the selected tiles. The loss is then back-propagated to the entire network, including the first CNN 40 and the aggregation operators.

In the training methods described above, the images correspond to the intended input images for the model (e.g. a histological section stained with hematoxylin and eosin stain) and each image is labelled depending on whether or not it corresponds to the specific biomarker that the model is to detect. However, the training methods may be modified to include transfer-learning from a related domain. In the case where it is not possible to acquire large annotated datasets, the models may be pre-trained on Task A (source), and then further trained on Task B (target), which only has limited annotated data at its disposal. Such training methods may be particularly of use in fields such as computational pathology, where annotations may involve a great cost of time and money, and may still be prone to errors related to subjectivity and experience. Furthermore, histopathological datasets in particular may contain at most a few thousand images. Thus pre-training the models on other computer vision datasets (e.g. from non medical fields) that are likely to contain a few million images may provide improved performance.

Different transfer learning strategies may be used to adapt a pre-trained model to another dataset, or to achieve higher generalisability by constraining the training with information coming from different sources.

It is possible to fine-tune the model, that is, to update the pre-trained weights using the target images. Instead of starting training from random weights, some pre-trained weights acquired from a different domain (such as computer vision) or from a different cancer dataset are used. Some of the layers are then frozen the weights are not updated further. Other layers are then further updated based on images which are labelled with the specific biomarker. While it is possible to fine-tune the whole model, the shallow layers are not updated in this example because they tend to learn the low-level features like edges and corners which are common in all images, whether they contain cars or cancer cells. Deeper layers, on the other hand, correspond to task-specific features, like cellular morphology, and hence are more likely to be updated using the target dataset.

It is also possible to use transfer learning by means of a different but related dataset as the source, such as a different type of cancer. For instance breast and colorectal cancers are both adenocarcinomas and have similar visual characteristics at the cellular level, making each other perfect candidates for being used in a transfer learning setting.

Transfer learning can also be considered within the context of domain adaptation, assuming that the source and target datasets are of a different but related distribution. Domain adaptation may deal with scenarios where a pre-trained model targets a new dataset with no labels, in which case, the labelled source dataset should be used to solve the new task in the target domain. Such a setting may be used for tasks dealing with multiple datasets, e.g. having breast cancer images obtained from different biobanks. The premise is to avoid the model learning only from a single source and improve its generalizability to other datasets, which may potentially not have any labelled data.

For instance, one scenario would be training a model for predicting molecular markers in dataset A and then applying it on images coming from dataset B. Even where both datasets are representative of the same type of cancer, e.g. breast, it is possible that the model would not perform as well on dataset B because tissue composition in WSIs are inherently diverse and there may exist differences in data due to using different scanners and slide preparation procedures while collecting the images. Domain adaptation aims to match the distributions of a target and source datasets within a shared space by transferring representations learnt in one domain to another.

In one example, a divergence-based domain adaptation technique is used to minimise a divergence criterion between the source and target data distributions, in order to learn a domain-invariant feature space. For instance a two-stream architecture (one for source, and one for target) can be trained jointly, while avoiding the weights diverging from each by using regularisation. An alternative domain adaptation techniques makes use of adversarial training with generator/discriminator models. In one example, generators are completely removed by introducing a domain confusion loss in order to teach the model how to discriminate images from different datasets and hence learn dataset-invariant features for better generalisability.

The domain adaptation problem may also be cast as a reconstruction task, to create a shared encoding representation for each of the domains while simultaneously learning to classify labelled source data, and to reconstruct the unlabelled target data. Alternatively, domain adaptation may be achieved by simultaneously training two generative adversarial networks that generate the images in the two respective domains. It can also be used in an offline setting to increase the number of images used for training by means of style transfer from source to target datasets. This naturally normalises the staining colors and styles of tissue images while preserving morphology.

In order to improve performance, data augmentation may additionally or alternatively be applied to a training dataset. This increases the generalisation capacity of the models. This may be particularly helpful in domains where data may be sparse, such as digital pathology.

A wide range of spatial and color transformations may be applied to images in the training dataset to create new training example images, to increase the variation in the data without the necessity of collecting new images. Example augmentation methods can be grouped in two sub-categories: linear transformations, such as rotation or flipping; and color spectrum augmentation, including brightness and contrast adjustment.

Since histopathological images are rotation-invariant, 90-degree rotations and horizontal/vertical flipping are used for populating the dataset without introducing any adverse effects. Color-based augmentation, on the other hand, makes the model learn beyond the original spectrum of brightness and contrast of the images, so that it can generalize better on images taken under different illumination. Non-linear transformations such as elastic nets may also be used, but may change the morphological composition of the tissue. Different augmentation methods may be combined and sequentially applied to an image.

Use of augmentation can have some side-effects if aggressively applied to a relatively small dataset, because the model is forced to learn not only the image features but also those introduced by augmentation. To mitigate this, augmentation may be applied whilst carrying out a hyper-parameter optimisation over 1) values of augmentation parameters and 2) combination of different parameter techniques and finding the subset of parameters and methods that improves the model's performance with respect to the case where no augmentation is used. Some probabilistic constraints may be applied to ensure that the model both sees the original images and the augmented ones during training.

In the examples described in Figures 5 and 6, a recurrent neural network (RNN) that can integrate the information from the tile level into the slide-level class probability by modelling the sequential dependency across tiles is used. End-to-end learning can additionally be provided by training the CNN and RNN module simultaneously.

In the examples described in Figures 6 and 7 a weighted average formulation, where weights are provided by an attention-based neural network 60, is used. Using an attention mechanism 60 also inherently gives insight towards the contribution of each tile to the final image prediction, and may potentially be used to highlight regions of interest that might be critical for computational pathology applications, without a priori annotations of regions in the image. The method is a deep-learning based weakly-supervised method that uses attention-based learning to identify regions with high diagnostic value for an accurate classification of whole slide images. Again, the attention module 60 may be training simultaneously with the CNN, and where present, the RNN module.

Both cases provide a fully differentiable and permutation-invariant means of aggregation. By permutation invariant, it is meant that no ordering or dependency is assumed for the tiles. The example described in relation to Figure 6 combines the advantages of RNNs and the attention mechanism. A cascaded model where the attention model is used for ranking the most representative tiles and the RNN for aggregating them is used to achieve the image-level prediction in this case.

Figure 10 above describes a method which directly incorporates the information from the negative class into the prediction capacity of the network, using a dual-branch architecture where each branch is responsible for a specific class, i.e. the positive branch 110 accounts for the positive class probabilities whereas the negative branch 120 focuses on the negative class. This model may be trained in different ways. In one example, the positive branch 110 and negative branch 120 are trained separately, in the manner described above. For the negative branch 120, the image labels will be 1 if the image does not correspond to the biomarker, and 0 if the image does correspond to the biomarker. The results may be combined by means of a linear or nonlinear function. Alternatively, the entire network may be trained simultaneously by back propagating the class-level loss to both branches.

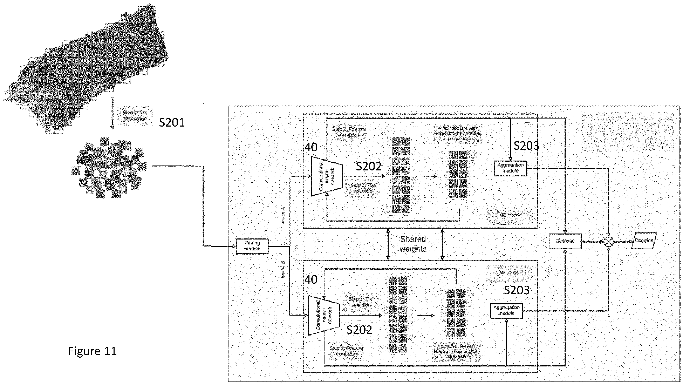

Figure 11 shows a schematic illustration of a method of training in accordance with an alternative embodiment. This method also aims to mitigate the class bias problem described in relation to Figure 10. The method uses a Siamese neural network structure. Siamese networks represent multiple instances of the same model with a shared architecture and weights.

In order to train the model, a contrastive loss function is used, such that the model learns the distance between positive and negative images alongside how to discriminate them. This is achieved by showing the model not only the tiles and labels, but also pairs of tiles with the same class label and pairs of different classes. The loss function then penalises the model anytime a large distance is computed for images of the same class and a small one for those from different classes. A pair of tiles is fed into to the first part of the first CNN 40 model, each tile input in a separate pass. The first CNN 50 outputs the d-dimensional feature vectors (also called embeddings) for each tile via two consecutive forward passes. The distance between the output vectors (embeddings) is then calculated, which forms the basis of the loss function. During training, it penalises the model anytime a large distance is computed for tiles of the same class or when the model thinks tiles of opposite classes are similar. For an image portion pair of $T_i$, $T_j$ and a label y, where y indicates the two images being from the same class (y=1) or from different classes (y=0) the loss is:

$$L(T_i, T_j, y) = (1 - y)L_s(D_w) + yL_d(D_w)$$

where the $L_s$ term is the loss computed for similar images and the $L_d$ term is the loss computed when the images are dissimilar. $D_w$ is the distance between two vectors and can be any distance (or similarity) function such as Euclidean distance or cosine similarity. When the terms are expanded the final loss may be given by:

$$(1 - y)\frac{1}{2}(D_W)^2 + (y)\frac{1}{2}\{\max(0, m - D_W)\}^2$$

where m is a margin.

Alternatively, the contrastive loss can be added to the cross entropy loss used by the profiler models as another regularising term. This way the model does not only learn how to identify positive images, but also is forced to learn the class-dependent characteristics of the domain which makes distinguishing a positive and negative class possible. In this case a regularised cross entropy loss in which the distance is incorporated as another term is used. In this case, two cross entropy (CE) losses are computed (through two forward passes), one for $T_i$ and one for $T_j$. The distance across their feature vectors is then computed to work out their distance (or similarity) using the aforementioned distance functions. The total loss is then:

$$L_{total} = L_{CE}(T_i, y_i) + L_{CE}(T_j, y_j) + wD_W(T_i, T_j)$$

where w is an optional weighting parameter, and $L_{CE}$ is the cross entropy loss described above.

As has been described above, the entire pipeline comprises a pre-processing module S201 that takes an image, e.g. a WSI, as input, subdivides it into a set of tiles, and streamlines these tiles through a series of neural networks comprising: 1) a deep convolutional neural network that is initially used for selecting the tiles that are representative of slides and later for feature extraction, 2) an attention-based neural network for identifying the important tiles for the prediction of molecular biomarkers, and/or 3) a recurrent neural network (RNN) for the aggregation of the selected tiles into a final image-level probability.

In the example described above, the input images are images of a histological section stained with hematoxylin and eosin stain, and the specific biomarker is a cancer biomarker which is a molecular biomarker, such as the ER biomarker, the HER2 biomarker, the PR biomarker, the EGFR biomarker or the MSI biomarker for example. As mentioned previously however, the antigen Ki-67 is also increasingly being tested as a marker for cell proliferation indicating cancer aggressiveness. Alternatively therefore, the specific biomarker may be Ki-67.

The reporting of Ki-67 is inherently discretised instead of binary categorical (i.e. whether a mutation/enrichment/expression exists on the tissue). Ki67 positivity may be defined as more than 10% of tumour cells staining positive for example, although the optimal cut-off threshold is still debatable. Identification of the KI67 index is inherently a different problem from predicting HR, ER, or HER2 profiles, as the outcome is a continuous value (i.e. percentage) rather than a discrete category. As a result, the problem cannot be straightforwardly cast as a MIL-problem, since the definition of positive or negative bags is invalid. However, using a predefined cut-off point to label the training data (e.g. a slide corresponding to greater than 10% is labelled 1, less than 10% is labelled 0), the problem can be cast as a binary classification, and models such as those described above in relation to Figures 4 to 7 may be used, and trained in the manner described above. The input to the model may be H&E stained slides, as described above. Additionally or alternatively, IHC image data may be used as input.

A methodology may be devised for the detection of nuclei in IHC images with Ki-67 staining, such that cell counting can be performed as a prerequisite to obtaining ground-truth Ki-67 scores. This is a manual step, performed to generate the labels for the H&E slides. In the example described above, the model is trained using images of a histological section stained with hematoxylin and eosin stain, each labelled as to whether the Ki-67 biomarker is present. The labels are determined from a corresponding IHC slide for example.

As described above in relation to Figure 3(c), a trained model M may be used in the image processing step S201 to perform cell segmentation. Such a model M is trained using ground-truth annotations. An expert annotator, such as a pathologist skilled in breast cancer, can delineate a subset of cells, which in turn can be used to train the model M to isolate cells from background as well as separate them from each other. The model M may be trained in an end-to-end fashion by using deep learning based encoder-decoder networks, in which images are first encoded into a low-dimensional feature space and then re-constructed to match their annotations, during which the model learns how to convert pixels into class labels, e.g. cell and background. The model M may be further modified by adding/dropping some network layers as well as by incorporating residual connections/blocks depending on the task at hand.

In some examples, the annotator directly interferes with the model output during training and corrects under- and/or over-segmentations. The expert-modified output is in turn submitted back to the model by means of external feedback to improve its performance.

Figure 3(e) is a schematic illustration of an example method of training a model M. The method trains the model to take input image data comprising a plurality of pixels and generate a value corresponding to each of the plurality of pixels, the values representing whether the pixel corresponds to cancer tissue. This model is trained in a separate training process.

In the figure, the input images are labelled I, the output from the model M is labelled O, the annotations provided by a human expert are labelled A, and a difference measure, or loss, is signified as D. The model M has an associated parameter vector θ. The parameters include the filter weights for all of the convolutional layers. The model M takes input images to create inferred annotations O corresponding to M(I, θ). The goal of the training process is to find a parameter vector θ' so that the difference between the annotations and the inferred annotations is minimised, i.e.

$$\theta' = argmin_\theta D(A, M(I, \theta))$$

M is the architecture of the network, while θ comprises the weights of the network. D represents a loss function. A pixel-wise cross entropy loss may be used. The Categorical Cross Entropy loss may be used. The pixel-wise loss is calculated as the log loss, summed over all possible categories C. In this case there are two categories, cancer tissue and non-cancer tissue. This is repeated over all pixels and averaged to give the loss. The pixel-wise loss is defined for each pixel at coordinate (x, y) as:

$$D_{x,y}(A_1, A_2) = -\sum_{i}^{C} t_i \log(f_i(s))$$

where $t_i$ is the correct annotation of a pixel taken from the annotation A for the category, and $f_i(s)$ the softmax function for the i-th category (out of a total C categories). The value t is equal to 1 for the correct category and 0 for the other categories, for each pixel. The vector of $t_i$ values for each pixel may be generated automatically from the annotated image. For i-th category, $t_i$ indicates whether a pixel is annotated as the i-th category, where $t_i$ =1 if the pixel is annotated as the category and 0 if not. The Softmax function $f_i(s)$ is given by:

$$f_i(s) = \frac{e^{S_i}}{\sum_{j}^{C} e^{S_j}}$$

where $S_j$ are the scores output by the final model layer for each category for the pixel. The loss then will be summed over every coordinate in the images.

The optimal parameters are computed by assigning random values as θ and then updating θ sequentially by computing the gradient of difference $\frac{\partial D}{\partial \theta}$ and updating θ with the computed gradient. The gradient of the loss with respect to each of the trainable parameters of the model is determined through back-propagation. The gradients are then used to determine the updated parameters, using an optimiser function. This family of update methods is known as gradient descent (GGD), generally defined iteratively as:

$$\theta = \theta - \mu \frac{\partial D}{\partial \theta}$$

where μ is the learning rate defining how quickly the parameters are updated. The update may be performed based on a batch average. A batch size of 8 tiles or 16 tiles is used for example.

An Adam optimization algorithm may be used. The optimisation strategy selected may depend on the performance of each strategy on a use-case however. For example, one of the following optimisation methods may be selected:

- Stochastic Gradient Descent (SGD)
- AdaDelta
- Adam
- AdaMax
- Nesterov Adam Optimiser
- RMSProp

The model is sensitive to pixel level annotations. In other words, if the training data were modified by just one pixel, parameters throughout the model may be updated differently. Including Atrous convolution filters of different sizes in a single layer in the model means that every pixel in the output is propagated from all around the input image. This means that a one-pixel difference can affect most parts of the neural network. This allows to update the model even with only one-pixel difference. Without using Atrous convolution, most changes may only be propagated locally.

The model is trained using data extracted from images annotated by human experts. Various other methods of training may also be used, for example using alterative loss functions. Once trained, the model is then used to process images that were not seen in training.

The approach described above for ER, PR, HER2 and Ki-67 can be applied across various cancer types and organs, including prediction of biomarkers modulated by commonly used cancer drugs and biomarkers that are relevant for cancer patient care.

Performance on various biomarkers are shown in Table 1 below. The models used are pre-trained on a dataset comprising 1.2 million images for a classification task including 1000 different categories. The models may then be further trained using a data set of cancer images, for example several thousand cancer images, and then further trained using a data set labelled with the specific biomarker, for example several hundred images. As shown, the methods show clinical-grade performance, i.e. 85% or higher. The Table 1 shows the performance metrics of the prediction on the biomarkers in the area under the curve (AUC) of the receiving operators characteristics (ROC) curve. When using normalized units, the area under the curve of the ROC curve is equal to the probability that the classifier will rank a randomly chosen positive instance higher than a randomly chosen negative one - in this case the probability that the model will output a higher probability for a randomly chosen image that is associated with the biomarker than a randomly chosen image that isn't associated with the biomarker.

| Biomarker | Performance (AUC, %) |
|-----------|----------------------|
| ER | 93% |
| PR | 94% |
| HER2 | 89% |
| MSI | 97% |
| EGFR | 85% |

Inclusion of the cancer cell segmentation stage described in relation to Figure 3 provided around a 3-7% better AUC for various receptors, when used together with an RNN aggregation operator, for both a default dataset and a cancer only dataset. Inclusion of the attention mechanism, in particular the method shown in relation to Figure 6, provided an improvement for HER2 of around 7% compared to the method shown in relation to Figure 3. Inclusion of an RNN based aggregation operator, in particular the method shown in relation to Figure 5, provided a 5-9% improvement in AUC for various receptors compared to the method shown in relation to Figure 3, using a default dataset.

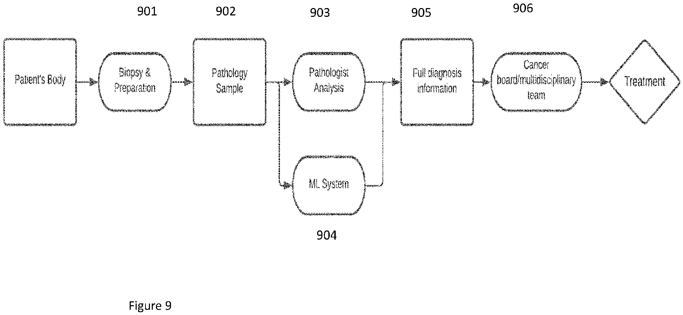

The methods described herein may provide clinical-grade instrument-free multi-cancer multi-markers profile prediction on histopathological tissue sample. Automatic profiling of biomarkers relevant for diagnostics, therapeutics and/or prognostics of cancer - including mutation status, receptor status, copy number variations etc - may be provided from whole slide H&E images, using a series of neural networks to identify correlations between cancer images and biomarker. The method is able to predict the outcome of biomarker tests at medical-grade level performance. The method may therefore replace the need for multiple tests. This may significantly streamline the diagnosis pipeline, as shown in Figure 9 for example.

Figure 9 shows an example diagnosis pipeline with automatic profiling of biomarkers. In step 901, a biopsy is performed, and a sample prepared in 902. The sample may be a tissue sample, stained with H&E. An image of the sample, is then analysed by a pathologist in 903. The image is also analysed by a machine learning based system such as the example described above in 904. The output of 903 and 904 is combined to give the full diagnosis information in 905, which is then provided to a cancer board or multidisciplinary team in 906. A treatment is then determined. By using the method described herein, operational and capital costs associated with the tests for biomarkers may be reduced. The diagnosis timeline may also be shortened by up to 97% - from up to 30 days to less than one day for example. The method may also simplify a pathologist's workflow by removing the need to revisit cases post-test, commissioning of tests, analyse test results etc. Finally, the method may reducing over- and under-diagnosis, as well as improve reproducibility.

The first and second models directly learn to discriminate positive and negative biomarker statuses by means of end-to-end MIL-based classification. Different aggregation methods have been described. The method may provide a deep-learning based framework to predict the clinical subtypes of breast cancer for example. The method may use end-to-end training with learnable aggregation functions and a tile selection procedure integrated to the model.

A list of example biomarkers is shown in Table 2 below:

Table 2: List of example molecular biomarkers

| Biomarkers | Cancer type/Primary site |
|------------|--------------------------|
| ABL1 | Blood/ bone marrow |
| ALK | Lung |
| AMER1 | Colon and rectum |

(continued)

| Biomarkers | Cancer type/Primary site |
|---|---|
| APC | Colon and rectum |
| ARID1A | Colon and rectum, pancreas, uterus |
| ATM | Prostate |
| BARD1 | Prostate |
| BRAF | Blood/Bone marrow, brain, skin, thyroid, lower GI tract, lung, colon and rectum |
| BRCA1 | Ovary, peritoneum, prostate, breast |
| BRCA2 | Ovary, peritoneum, prostate, breast |
| BRIP1 | Prostate |
| CASP8 | Cervix |
| CD274 | Cervix, colon and rectum, lung, stomach |
| CDK12 | Prostate |
| CDKN21 | Kidney |
| CDKN2A | Head and neck, kidney, lung, pancreas, bladder |
| CHEK1 | Prostate |
| CHEK2 | Prostate |
| CMTR2 | Lung |
| CTNNB1 | Uterus |
| DOT1 L | Lung |
| E2F1 | Head and neck |
| EEF1A1 | Liver |
| EGFR | Lung |
| EML | Thyroid |
| ER | Breast |
| ERBB2 | Lung, esophagus, lower GI tract, uterus, breast, stomach, colon and rectum, stomach |
| ERBB3 | Cervix |
| ERG | Prostate |
| EZH2 | Lymph node |
| FANCL | Prostate |
| FGFR2 | Urinary bladder, bile duct, lung |
| FGFR3 | Bladder |
| FLCN | Kidney |
| FLI1 | Prostate |
| FLT3 | Blood/ bone marrow |
| FOXA1 | Prostate, breast |
| GATA3 | Breast |
| GATA6 | Pancreas |
| HER2 | Breast, stomach |
| HLA-A | Cervix |

(continued)

| Biomarkers | Cancer type/Primary site |
|---|---|
| HRAS | Thyroid, head and neck |
| IDH1 | Blood/ bone marrow, prostate |
| IDH2 | Blood/ bone marrow |
| IGF2 | Lower GI tract, colon and rectum |
| JAK2 | Stomach, colon and rectum |
| Ki67 | Breast |
| KIT | GI tract, skin, thymus, colon and rectum, stomach |
| KRAS | Colon and rectum, lower GI tract, thyroid, pancreas, uterus, stomach |
| LZTR1 | Liver |
| MAP3K1 | Breast |
| MDM2 | Bladder |
| MET | Lung, kidney |
| MLH1 | Colon |
| MSH2 | Colon |
| MSH6 | Colon |
| MSI | Colon and rectum, stomach |
| NF1 | Ovary, cervix |
| NOTCH 1 | Head and neck, lung |
| NOTCH2 | Head and neck |
| NRAS | Lower GI tract, thyroid, colon and rectum |
| NTRK1 | All solid tumors |
| NTRK2 | All solid tumors |
| NTRK3 | All solid tumors |
| P53 | Ovary |
| PALB2 | Prostate |
| PDCD1LG2 | Cervix, colon and rectum, stomach |
| PDGFRA | GI tract, blood/bone marrow, colon and rectum, stomach |
| PDL1 | Lung, stomach, cervix |
| PDL2 | Cervix, stomach |
| PI(3)K/ AKT pathway | Uterus |
| PIK3CA | Head and neck, breast, colon and rectum, stomach |
| PMS2 | Colon |
| POLE | Lower GI tract, uterus, colon and rectum, uterus |
| PPP3CA | Lung |
| PR | Breast |
| PTEN | Uterus, kidney |
| RAD51B | Prostate |
| RAD51C | Prostate |

(continued)

| Biomarkers | Cancer type/Primary site |
| --- | --- |
| RAD51 D | Prostate |
| RAD54L | Prostate |
| RASA1 | Lung |
| RB1 | Ovary, breast, cervix, liver |
| RET | Thyroid, lung |
| ROS | Lung |
| SF3B1 | Liver |
| SHKBP1 | Cervix |
| SMAD2 | Colon and rectum |
| SMAD3 | Lower Gi tract |
| SMAD4 | Pancreas, lower Gi tract |
| SMAD4 | Colon and rectum |
| SMARCA4 | Liver |
| SMARCB1 | Sarcoma |
| SOX2 | Esophagus, lung |
| SOX9 | Lower GI tract, colon and rectum |
| SPOP | Prostate |
| TFE3 | Kidney |
| TGFBR2 | Cervix |
| TP53 | Breast, colon and rectum, lung, uterus, bladder, kidney, pancreas, head and neck, liver, ovary |
| TP63 | Esophagus |
| TRAF3 | Head and neck |
| VEGFA | Esophagus |
| VHL | Kidney |
| MTOR | Stomach |
| KMT2B | Colon and rectum, stomach |
| FBXW7 | Lung, stomach |
| KEAP1 | Lung |
| KMT2C | Stomach |
| KMT2D | Colon and rectum, stomach |
| MAP2K4 | Breast |
| MGA | Colon and rectum |
| PBRM1 | Stomach |
| PDGFRB | Lung |
| PIK3R1 | Cervix, lung |
| PPP6C | Skin |
| PTCH1 | Colon and rectum |

(continued)

| Biomarkers | Cancer type/Primary site |
| --- | --- |
| RHOA | Head and neck |
| RNF43 | Colon and rectum, stomach |
| RREB1 | Stomach |
| SETD2 | Kidney |
| STK11 | Cervix |
| TCERG1 | Cervix |

While certain embodiments have been described, these embodiments have been presented by way of example only, and are not intended to limit the scope of the inventions. Indeed the novel methods and apparatus described herein may be embodied in a variety of other forms; furthermore, various omissions, substitutions and changes in the form of methods and apparatus described herein may be made.

**Claims**

1. A computer implemented method of processing an image of tissue, comprising:

   obtaining a first set of image portions from an input image of tissue;
   selecting a second set of one or more image portions from the first set of image portions, the selecting comprising inputting image data of an image portion from the first set into a first trained model comprising a first convolutional neural network, the first trained model generating an indication of whether the image portion is associated with a biomarker; and
   determining an indication of whether the input image is associated with the biomarker from the second set of one or more image portions.

2. The method of claim 1, wherein the second set comprises two or more image portions, and wherein the determining comprises inputting first data corresponding to the second set of one or more image portions into a second trained model.

3. The method of claim 2, wherein the second trained model comprises a recurrent neural network.

4. The method of claim 2 or 3, wherein the second trained model comprises an attention mechanism.

5. The method of claim 3, wherein the second trained model further comprises an attention mechanism, and wherein determining an indication of whether the input image is associated with the biomarker from the second set of image portions comprises:

   inputting the first data for each image portion in the second set into the attention mechanism, wherein the attention mechanism is configured to output an indication of the importance of each image portion;
   selecting a third set of image portions based on the indication of the importance of each image portion;
   for each image portion in the third set, inputting the first data into the recurrent neural network, the recurrent neural network generating the indication of whether the input image is associated with the biomarker.

6. The method of any of claims 2 to 5, wherein the indication of whether the image portion is associated with the biomarker is a probability that the image portion is associated with the biomarker, wherein selecting the second set comprises selecting the k image portions having the highest probability, wherein k is a pre-defined integer greater than 1.

7. The method of any of claims 2 to 5, wherein the first convolutional neural network comprises a first portion comprising at least one convolutional layer and a second portion, wherein the second portion takes as input a one dimensional vector;
   wherein determining the indication of whether the input image is associated with the biomarker from the second set of image portions further comprises:
generating the first data for each of the second set of image portions, generating the first data for an image portion comprising inputting the image data of the image portion into the first portion of the first convolutional neural network.

8. The method according to any preceding claim, further comprising:

selecting a fourth set of one or more image portions from the first set of image portions, the selecting comprising inputting image data of an image portion from the first set into a third trained model comprising a second convolutional neural network;
wherein the indication of whether the input image is associated with the biomarker is determined from the fourth set of one or more image portions and the second set of one or more image portions.

9. The method of any preceding claim, wherein the biomarker is a cancer biomarker and wherein obtaining the first set of image portions from an input image of tissue comprises:

splitting the input image of tissue into image portions;
inputting image data of an image portion into a fifth trained model, the fifth trained model generating an indication of whether the image portion is associated with cancer tissue;
selecting the first set of image portions based on the indication of whether the image portion is associated with cancer tissue.

10. The method of any preceding claim, wherein the biomarker is a molecular biomarker.

11. A system for processing an image of tissue, comprising:

an input configured to receive an input image of tissue;
an output configured to output an indication of whether the input image is associated with a biomarker
one or more processors, configured to:

obtain a first set of image portions from an input image of tissue received by way of the input;
select a second set of one or more image portions from the first set of image portions, the selecting comprising inputting image data of an image portion from the first set into a first trained model comprising a first convolutional neural network, the first trained model generating an indication of whether the image portion is associated with a biomarker;

determine an indication of whether the input image is associated with the biomarker from the second set of one or more image portions; and
output the indication by way of the output.

12. A computer implemented method of training, comprising:

obtaining a first set of image portions from an input image of tissue;
inputting image data of an image portion from the first set into a first model comprising a first convolutional neural network, the first model generating an indication of whether the image portion is associated with a biomarker;
adapting the first model based on a label associated with the input image of tissue indicating whether the input image is associated with the biomarker.

13. A method according to claim 12, further comprising:

selecting a second set of one or more image portions from the first set of image portions based on the indication of whether the image portion is associated with a biomarker;
determining an indication of whether the input image is associated with the biomarker from the second set of one or more image portions by inputting first data corresponding to the second set of image portions into a second model, and wherein the method further comprises adapting the second model based on the label associated with the input image of tissue indicating whether the input image is associated with the biomarker.

14. A system comprising a first model and a second model trained according to the method of claim 12 or 13.

15. A carrier medium comprising computer readable code configured to cause a computer to perform the method of any of claims 1 to 10 or 12 to 13.

1

11

Input

Processor

3

Output

13

9

Model

Storage

5

7

Figure 1

Image pre-processing    S201

Selecting first set of image sections(s)    S202

Determining indication of biomarker    S203

Figure 2(a)

Figure 2(b)

Figure 3(a)

Input image I

S201 –
Image pre-processing

First set of image
portions

Image splitting    S301

Background detection    S302

Filter    S303

Segmentation    S304

Figure 3(b)

Figure 3(c)

Layer n

Output layer n-1

output m

layer m

output l

layer l

output k

layer k

input

Acausal convolution

Concatenate

Elementwise

output n

layer o

output o

layer p

output p

max pooling

layer q

output q

layer r

output

skip connection

Figure 3(d)

Figure 3(e)

Figure 4

I

S201

40

Convolutional
neural
network

Top-ranked tile,
representing the whole
slide.

Figure 5(a)

Figure 5(b)

Figure 5(c)

Figure 6(a)

S201

40

S202

Step 1: Tile
selection

k selected tiles with
respect to their positive
probability

Step 2: Feature
extraction

60 | Attention
module

Decision

Step 4:
Attention-based
aggregation

S203

d

k

Fully connected layer 1

60

Fully connected layer 2

k

Figure 6(b)

Figure 7

Figure 8

901 902 903 905 906

Patient's Body → Biopsy & Preparation → Pathology Sample → Pathologist Analysis → Full diagnosis information → Cancer board/multidisciplinary team → Treatment

ML System

904

Figure 9

Figure 10

Figure 11

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2019/183596 A1 (MEMORIAL SLOAN KETTERING CANCER CENTER [US]) 26 September 2019 (2019-09-26) | 1-4,6-9, 11-15 | INV. G06T7/00 |
| A | * Fig. 3. Fig. 33C. [0202,0204,0206,0207][0183-187,193][0197] Fig. 19. [0073-0077] and corresponding Fig. 3 [0156, 0162] * | 5,10 | |
| X | US 2019/206056 A1 (GEORGESCU WALTER [US] ET AL) 4 July 2019 (2019-07-04) * [0001,4-10][0035] Fig. 5, paragraph [0135,137,138,139] * | 1,8-11 | |
| X | US 2020/250398 A1 (COURTIOL PIERRE [FR] ET AL) 6 August 2020 (2020-08-06) * [0006][0076-78][0028] Fig. 1 * | 1,8,9,11 | |
| A | US 2020/123618 A1 (BATENCHUK CORY [US] ET AL) 23 April 2020 (2020-04-23) * [0007,0051,0066,0069] * | 1-15 | TECHNICAL FIELDS SEARCHED (IPC) G06T |

The present search report has been drawn up for all claims

| Place of search | Date of completion of the search | Examiner |
|---|---|---|
| Munich | 9 March 2021 | Nicolau, Stephane |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

**ANNEX TO THE EUROPEAN SEARCH REPORT
ON EUROPEAN PATENT APPLICATION NO.**                       EP 20 19 8551

This annex lists the patent family members relating to the patent documents cited in the above-mentioned European search report.
The members are as contained in the European Patent Office EDP file on
The European Patent Office is in no way liable for these particulars which are merely given for the purpose of information.

09-03-2021

| Patent document cited in search report | | Publication date | Patent family member(s) | | Publication date |
|---|---|---|---|---|---|
| WO 2019183596 | A1 | 26-09-2019 | EP | 3769282 A1 | 27-01-2021 |
| | | | US | 2019295252 A1 | 26-09-2019 |
| | | | US | 2020043164 A1 | 06-02-2020 |
| | | | US | 2021049763 A1 | 18-02-2021 |
| | | | WO | 2019183596 A1 | 26-09-2019 |
| US 2019206056 | A1 | 04-07-2019 | AU | 2018394106 A1 | 16-07-2020 |
| | | | CN | 111542830 A | 14-08-2020 |
| | | | EP | 3625765 A2 | 25-03-2020 |
| | | | US | 2019206056 A1 | 04-07-2019 |
| | | | US | 2020364867 A1 | 19-11-2020 |
| | | | WO | 2019133538 A2 | 04-07-2019 |
| US 2020250398 | A1 | 06-08-2020 | NONE | | |
| US 2020123618 | A1 | 23-04-2020 | US | 2020123618 A1 | 23-04-2020 |
| | | | WO | 2020081463 A1 | 23-04-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description

- **MACENKO et al.** A method for normalizing histology slides for quantitative analysis. *IEEE International Symposium on Biomedical Imaging: From Nano to Macro,* 2009